(19) Europäisches Patentamt
European Patent Office
Office européen des brevets

(11) **EP 4 712 085 A1**

(12) **EUROPEAN PATENT APPLICATION**

(43) Date of publication:
**18.03.2026 Bulletin 2026/12**

(21) Application number: **25199592.4**

(22) Date of filing: **02.09.2025**

(51) International Patent Classification (IPC):
**G16B 40/10** (2019.01)   **G16B 45/00** (2019.01)
**G16C 20/40** (2019.01)   **G16C 20/80** (2019.01)

(52) Cooperative Patent Classification (CPC):
**G16B 45/00; G16B 40/10; G16C 20/40;
G16C 20/70; G16C 20/80**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.09.2024 US 202463692376 P**

(71) Applicant: **HighChem s.r.o.
821 09 Bratislava (SK)**

(72) Inventor: **BODNÁR, Gergo
82109 Bratislava (SK)**

(74) Representative: **Boult Wade Tennant LLP
Salisbury Square House
8 Salisbury Square
London EC4Y 8AP (GB)**

(54) **MOLECULAR NETWORK FOR LIBRARY MOLECULAR STRUCTURAL CONTENT**

(57)   Embodiments described herein relate to a process for molecular network generation based on molecular structural content. A system can comprise a memory that stores, and a processor that executes, computer executable components. The computer executable components can comprise an evaluating component that executes a comparison of a first molecular fingerprint, comprising first molecular structure data of a first molecular structure, to a second molecular fingerprint, comprising second molecular structure data of a second molecular structure, and a visualizing component that generates display data for visualizing a similarity visual illustrating representations of the first molecular structure, the second molecular structure, and a structural similarity score resulting from the comparison. The representations can comprise an edge, corresponding to the structural similarity score, extending between a pair of nodes, corresponding to the first molecular structure and the second molecular structure.

SCIENTIFIC INSTRUMENT MODULE 100

FIRST (MOLECULAR STRUCTURE DATA IDENTIFICATION) LOGIC 102

SECOND (COMPARING) LOGIC 104

THIRD (DISPLAYING) LOGIC 106

FOURTH (FILTERING) LOGIC 108

**FIG. 1**

EP 4 712 085 A1

**Description**

BACKGROUND

A molecular network can be employed to provide interpretation of chemically similar compounds in a chemical space network. Such molecular network can be used to address a high capacity of library content that increases over time.

BRIEF DESCRIPTION OF THE DRAWINGS

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.

FIG. 1 illustrates a block diagram of an example scientific instrument for performing one or more operations, in accordance with one or more example embodiments described herein.

FIG. 2 illustrates a flow diagram of an example method of performing operations using the scientific instrument of FIG. 1, in accordance with one or more example embodiments described herein.

FIG. 3 illustrates a graphical user interface (GUI) that can be used in the performance of one or more of the methods described herein, in accordance with one or more example embodiments described herein.

FIG. 4 illustrates a block diagram of an example computing device that can perform one or more of the methods disclosed herein, in accordance with one or more example embodiments described herein.

FIG. 5 illustrates a block diagram of an example, non-limiting system that can facilitate a process for molecular network generation and/or visualization, in accordance with one or more example embodiments described herein.

FIG. 6 illustrates a block diagram of another example, non-limiting system that can facilitate a process for molecular network generation and/or visualization, in accordance with one or more example embodiments described herein.

FIG. 7 illustrates an example molecular fingerprint visualization, in accordance with one or more example embodiments described herein.

FIG. 8 illustrates an example set of molecular structural content of a library data store, in accordance with one or more example embodiments described herein.

FIG. 9 illustrates an example visualization of a molecular network, in accordance with one or more example embodiments described herein.

FIG. 10 illustrates another example visualization of a molecular network, in accordance with one or more example embodiments described herein.

FIG. 11 illustrates yet another example visualization of a molecular network, in accordance with one or more example embodiments described herein.

FIG. 12 illustrates still another example visualization of a molecular network, in accordance with one or more example embodiments described herein.

FIG. 13 illustrates a diagram of an interactive GUI that can be employed to customize one or more parameters employed by the one or more example embodiments described herein to generate a molecular network visualization, in accordance with one or more example embodiments described herein.

FIG. 14 illustrates a flow diagram of one or more processes that can be performed by the molecular network generation system of FIG. 5, in accordance with one or more example embodiments described herein.

FIG. 15 illustrates another flow diagram of one or more processes that can be performed by the molecular network generation system of FIG. 6, in accordance with one or more example embodiments described herein.

FIG. 16 illustrates a continuation of the flow diagram of FIG. 15 of one or more processes that can be performed by the molecular network generation system of FIG. 6, in accordance with one or more example embodiments described herein.

FIG. 17 illustrates a block diagram of example scientific instrument system in which one or more of the methods described herein can be performed, in accordance with one or more example embodiments described herein.

FIG. 18 illustrates a block diagram of an example operating environment into which embodiments of the subject matter described herein can be incorporated.

FIG. 19 illustrates an example schematic block diagram of a computing environment with which the subject matter described herein can interact and/or be implemented at least in part.

SUMMARY

The following presents a summary to provide a basic understanding of one or more example embodiments described herein. This summary is not intended to identify key or critical elements, and/or to delineate scope of particular embodiments or scope of claims. Its sole purpose is to present concepts in a simplified form as a prelude to the more detailed description that is presented later. In one or more example embodiments, systems, computer-implemented methods, apparatuses and/or computer program products described herein can provide a plug-and-play process for generating, visualizing and/or employing a molecular network for various databases of molecular structure data using a visualization framework.

In accordance with an embodiment, a system can comprise a memory that stores computer executable components, and a processor that executes the computer executable components. The computer executable components can comprise an evaluating component that executes a comparison of a first molecular fingerprint, comprising first molecular structure data of a first molecular structure, to a second molecular fingerprint, comprising second molecular structure data of a second molecular structure; and a visualizing component that generates display data for visualizing a similarity visual illustrating representations of the first molecular structure, the second molecular structure, and a structural similarity score resulting from the comparison.

In accordance with another embodiment, a computer-implemented method can comprise executing, by a system operatively coupled to a processor, a comparison of a first molecular fingerprint, comprising first molecular structure data of a first molecular structure, to a second molecular fingerprint, comprising second molecular structure data of a second molecular structure; and generating, by the system, display data for visualizing a similarity visual illustrating representations of the first molecular structure, the second molecular structure, and a structural similarity score resulting from the comparison.

In accordance with still another embodiment, a computer program product facilitates a process for visualizing and comparing molecular structures, the program instructions executable by a processor to cause the processor to execute, by the processor, a comparison of a first molecular fingerprint, comprising first molecular structure data of a first molecular structure, to a second molecular fingerprint, comprising second molecular structure data of a second molecular structure; and generate, by the processor, display data for visualizing a similarity visual illustrating representations of the first molecular structure, the second molecular structure, and a structural similarity score resulting from the comparison.

The one or more example embodiments described herein can be implemented within, in connection with and/or coupled to a scientific imaging device.

The one or more example embodiments disclosed herein can be applied on a plug-and-play basis to various architectures of existing molecular structural library and/or library datastores of molecular structural data. That is, the one or more example embodiments described herein can generate a molecular network comprising a visual representing a plurality of chemical relationships regardless of data structure of a molecular structural library.

The one or more example embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary illustrated chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

That is, the one or more example embodiments described herein can be employed to generate a molecular network that can provide varying outputs during use of the molecular network. For example, one or more visual aspects of a format of a MN cloud, such as coloring, line thicknesses, shapes and/or distances between different aspects of the MN cloud can be generated that can be employed by a user entity to predict one or more chemical properties and/or relationships corresponding illustrated nodes. These one or more chemical properties and/or relationships can comprise chemical class, chemical use, similar compounds, etc.

Further aspects of the invention are set out in the following numbered clauses:

Clause 1. A system, comprising:

a memory that stores computer executable components; and
a processor that executes the computer executable components stored in the memory, wherein the computer executable components comprise:

an evaluating component that executes a comparison of a first molecular fingerprint, comprising first molecular structure data of a first molecular structure, to a second molecular fingerprint, comprising second molecular structure data of a second molecular structure; and
a visualizing component that generates display data for visualizing a similarity visual illustrating representations of the first molecular structure, the second molecular structure, and a structural similarity score resulting from the comparison.

Clause 2. The system of clause 1, wherein the representations comprise an edge, corresponding to the structural similarity score, extending between a pair of nodes, corresponding to the first molecular structure and the second molecular structure.

Clause 3. The system of clause 2, wherein the computer executable components further comprise:
a parameterizing component that applies a first property of the structural similarity score as a first visual modification of the edge and that applies a second property of the first molecular structure as a second visual modification of the respective node of the first molecular structure.

Clause 4. The system of clause 1, wherein the computer executable components further comprise:
a scoring component that generates the structural similarity score based on sub-comparisons of first fingerprint bits that are common to each of the first molecular fingerprint and the second molecular fingerprint, and of second fingerprint bits that are uncommon, to each of the first molecular fingerprint and the second molecular fingerprint.

Clause 5. The system of clause 1, wherein the computer executable components further comprise:

a fingerprinting component that generates the first molecular fingerprint based on the first molecular structure,
wherein the first molecular fingerprint is unique to the first molecular structure and has identification metadata, unique to the first molecular fingerprint, associated therewith.

Clause 6. The system of clause 1, wherein the computer executable components further comprise:
a displaying component that evaluates identification metadata associated with the first molecular fingerprint or the second molecular fingerprint and generates, based on the evaluating, the similarity visual comprising a visualization aspect that visually differentiates nodes, corresponding to the first molecular fingerprint or the second molecular fingerprint, from one another.

Clause 7. The system of clause 1, wherein the computer executable components further comprise:
a displaying component that generates the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

Clause 8. The system of clause 7, wherein the computer executable components further comprise:

a filtering component that redistributes a portion of the set of nodes, including the primary node, based on selection, at a graphical user interface displaying the similarity visual, of a classification filtering option corresponding to the first molecular structure,
wherein the scoring component generates a set of similarity scores between nodes of the portion of the set of nodes resulting from the filtering.

Clause 9. The system of clause 1,
wherein the similarity visual comprises a two-dimensional representation of the first molecular structure and the second molecular structure that are moveable relative to one another and size adjustable relative to one another.

Clause 10. A computer-implemented method, comprising:

executing, by a system operatively coupled to a processor, a comparison of a first molecular fingerprint, comprising first molecular structure data of a first molecular structure, to a second molecular fingerprint, comprising second molecular structure data of a second molecular structure; and
generating, by the system, display data for visualizing a similarity visual illustrating representations of the first molecular structure, the second molecular structure, and a structural similarity score resulting from the comparison.

Clause 11. The computer-implemented method of clause 10, wherein the representations comprise an edge, corresponding to the structural similarity score, extending between a pair of nodes, corresponding to the first molecular structure and the second molecular structure.

Clause 12. The computer-implemented method of clause 10, further comprising:
applying, by the system, a first property of the structural similarity score as a first visual modification of the edge and that applies a second property of the first molecular structure as a second visual modification of the respective node of the first molecular structure.

Clause 13. The computer-implemented method of clause 10, further comprising:

evaluating, by the system, identification metadata associated with the first molecular fingerprint or the second molecular fingerprint; and
generating, by the system, based on the evaluating, the similarity visual comprising a visualization aspect that visually differentiates nodes, corresponding to the first molecular fingerprint or the second molecular fingerprint, from one another.

Clause 14. The computer-implemented method of clause 10, further comprising:
generating, by the system, the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

Clause 15. The computer-implemented method of clause 14, further comprising:

redistributing, by the system, a portion of the set of nodes, including the primary node, based on selection, at a graphical user interface displaying the similarity visual, of a classification filtering option corresponding to the first molecular structure; and
generating, by the system, a set of similarity scores between nodes of the portion of the set of nodes resulting from the filtering.

Clause 16. A computer program product facilitating a process for visualizing and comparing molecular structures, the computer program product comprising a computer readable storage medium having program instructions embodied therewith, and the program instructions executable by a processor to cause the processor to:

execute, by the processor, a comparison of a first molecular fingerprint, comprising first molecular structure data of a first molecular structure, to a second molecular fingerprint, comprising second molecular structure data of a second molecular structure; and
generate, by the processor, display data for visualizing a similarity visual illustrating representations of the first molecular structure, the second molecular structure, and a structural similarity score resulting from the comparison.

Clause 17. The computer program product of clause 16, wherein the representations comprise an edge, corresponding to the structural similarity score, extending between a pair of nodes, corresponding to the first molecular structure and the second molecular structure.

Clause 18. The computer program product of clause 16, wherein the program instructions are further executable by the processor to cause the processor to:

evaluate, by the processor, identification metadata associated with the first molecular fingerprint or the second molecular fingerprint; and
generate, by the processor, based on the evaluating, the similarity visual comprising a visualization aspect that visually differentiates nodes, corresponding to the first molecular fingerprint or the second molecular fingerprint, from one another.

Clause 19. The computer program product of clause 16, wherein the program instructions are further executable by the processor to cause the processor to:
generate, by the processor, the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

Clause 20. The computer program product of clause 19, wherein the program instructions are further executable by the processor to cause the processor to:

redistribute, by the processor, a portion of the set of nodes, including the primary node, based on selection, at a graphical user interface displaying the similarity visual, of a classification filtering option corresponding to the first molecular structure; and
generate, by the processor, a set of similarity scores between nodes of the portion of the set of nodes resulting from the filtering.

DETAILED DESCRIPTION

The following detailed description is merely illustrative and is not intended to limit embodiments and/or application or utilization of embodiments. Furthermore, there is no intention to be bound by any expressed or implied information presented in the preceding Summary section, or in the Detailed Description section. One or more example embodiments are now described with reference to the drawings, wherein like reference numerals are utilized to refer to like elements throughout. In the following description, for purposes of explanation, numerous specific details are set forth in order to provide a more thorough understanding of the one or more example embodiments. It is evident, however, in various cases, that the one or more example embodiments can be practiced without these specific details.

Various operations can be described as multiple discrete actions or operations in turn, in a manner that is most helpful in understanding the subject matter disclosed herein. However, the order of description should not be construed as to imply that these operations are necessarily order dependent. In particular, these operations can be performed in an order different from the order of presentation. Operations described can be performed in a different order from the described embodiment. Various additional operations can be performed, and/or described operations can be omitted in additional embodiments.

Turning now to the subject of molecular networking, molecular networking can organize molecular structural data into a network, such as a relational molecular structural network, thereby mapping the chemistry behind molecules (e.g., based on the ions of the molecular structure, bonds of the molecular structure, etc.).

In existing frameworks, such molecular networking can be employed at most in a two-dimensional, non-customizable format. Indeed, existing frameworks can illustrate molecular structure or molecular fingerprints and may provide lists of data illustrating aggregated quantities of similar fingerprint bits (e.g., ions or bonds). However, analysis of a large and growing library of molecular structures for similarities, patterns, groupings etc. is not possible by such listings. Indeed, there is no visualization that can aid a user for a rapid analysis when performing an experiment, operating a test, performing a manufacturing operation, etc. Indeed, with existing frameworks, limited output can be obtained ahead of time via extensive evaluation (e.g., extensive in terms of time, labor, power, etc.).

To account for one or more deficiencies of such existing frameworks, one or more example embodiments are described herein that can provide rapid increase and efficiency of recognition and/or illustration of a chemical relationships between molecular structures, which can add increased value to use of and generation of a molecular network (MN). In one or more cases, one or more example embodiments described herein can allow for generation and display of a dynamically adjustable cloud-based MN visualization to allow for a plurality of customized visuals for use by a user entity in varying in-process operations (e.g., experiments, tests, manufacturing operations, etc.).

Generally, the one or more example embodiments described herein can provide generation of a molecular network based on a molecular structural library, updating of the molecular structural library based on a new molecular structure data, and/or generation of a dynamically-adjustable and customizable molecular network cloud visual.

The one or more example embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

For example, one or more molecular networking application embodiments as described herein can aid in determining varying types of chemical relationships and/or molecular structure relationships between molecular structures. In one or more cases, such one or more example embodiments can enhance understanding of structural similarities between query and library through visualization of nodes and edges that can comprise different metadata available in various libraries and/or library types.

That is, the one or more example embodiments described herein can be employed to generate a molecular network that can provide varying outputs during use of the molecular network. For example, one or more visual aspects of a format of a MN cloud, such as coloring, line thicknesses, shapes and/or distances between different aspects of the MN cloud can be generated that can be employed by a user entity to predict one or more chemical properties and/or relationships corresponding illustrated nodes. These one or more chemical properties and/or relationships can comprise chemical class, chemical use, similar compounds, etc.

Further, regarding functioning of the one or more example embodiments described herein, such can be implemented within, in connection with and/or coupled to a scientific imaging device. This implementation can be applied on a plug-and-play basis to various architectures of existing molecular structural library and/or library datastores of molecular structural data. That is, the one or more example embodiments described herein can generate a molecular network comprising a visual representing a plurality of chemical relationships regardless of data structure of a molecular structural library.

Discussion next turns to a general discussion of one or more scientific instrument systems disclosed herein, as well as to related methods, computing devices, and/or computer-readable media. For example, in one or more example embodiments, a system can comprise a memory that stores computer executable components and a processor that executes the computer executable components stored in the memory. The computer executable components can comprise an evaluating component that executes a comparison of a first molecular fingerprint, comprising first molecular structure data of a first molecular structure, to a second molecular fingerprint, comprising second molecular structure data of a second molecular structure. The computer executable components also can comprise a visualizing component that generates display data for visualizing a similarity visual illustrating representations of the first molecular structure, the second molecular structure, and a structural similarity score resulting from the comparison.

The embodiments disclosed herein thus can provide improvements to scientific instrument technology (e.g., improvements in the computer technology supporting such scientific instruments, among other improvements), which can be employed in various fields including optics, signal processing, spectroscopy, and/or nuclear magnetic resonance (NMR), without being limited thereto.

Various ones of the embodiments disclosed herein can improve upon existing approaches to achieve the technical advantages of high information and/or accurate information molecular network generation, visualization and/or operation (e.g., use of the MN). That is, the one or more frameworks described herein can provide a more accurate construction, as compared to existing frameworks, of a molecular network and/or molecular network cloud visual based on the MN, thereby allowing for identification of a chemical property, chemical relationship, and/or chemical classification. This identification can be performed by a user entity based an MN generated and/or on another visual representation of the corresponding MN cloud. The molecular structures being evaluated (e.g., the molecular structure data defining the molecular structures) can arise from any suitable source, such as from a scientific imaging device source using any suitable method, such as electron holography imaging, scanning electron microscope (SEM) imaging, electron microscope (EM) imaging, and/or the like.

Such technical advantages are not achievable by routine and/or existing approaches, as described above, and all user entities of systems including such embodiments can benefit from these advantages (e.g., by assisting the user entity in the performance of a technical task, such as identification of one or more molecular structure relationships, by means of molecular network generation, molecular network visualization, and/or molecular network operation.

The technical features of the embodiments disclosed herein (e.g., analysis of data defining molecular structures and comparison of molecular structures to determine relationships thereof) are thus decidedly unconventional in the field of material analysis, in addition to the fields of optics, signal processing, spectroscopy, and/or NMR, without being limited thereto, as are combinations of the features of the embodiments disclosed herein.

As discussed further herein, various aspects of the embodiments disclosed herein can improve the functionality of a computer itself. That is, the computational and/or user interface features disclosed herein do not involve only the collection and/or comparison of information but instead can apply new analytical and technical techniques to change the operation of the computer-analysis of material compounds. For example, based on the use of fingerprinting, structural similarity scoring, and virtual representation of molecular structural content, a MN having greater accuracy, greater customization, and/or dynamic flexibility (e.g., parameterization, filtering, etc.) can be provided, as compared to existing frameworks. As a result thereof, use of a MN and/or of a MN visualization (e.g., MN cloud visual) can be accompanied by an increase in speed and/or accuracy of response related to a query. As such, one or more non-limiting systems described herein, comprising a molecular network generation system, can be self-improving.

The present disclosure thus introduces functionality that neither an existing computing device, nor a human, could perform. Rather, such existing computing devices are ineffective at generation of molecular networks, relationships are poorly represented or not represented at all, and/or the examination of long tables can be a difficult, error-prone and/or time-consuming task in view of compounded errors and poor relationship representation, thereby resulting in loss of accuracy, efficiency and/or speed when evaluating molecular structures and relationships therebetween. This can particularly be the case when evaluating large libraries of molecular structures. In view of the time, energy and/or loss of data involved, it is not practical to operate within the confines of existing approaches.

Accordingly, the embodiments of the present disclosure can serve any of a number of technical purposes, such as controlling a specific technical system or process; determining from measurements how to control a machine; digital audio, image, or video enhancement or analysis; separation of material sources in a mixed signal; generating data for reliable and/or efficient transmission or storage; providing estimates and confidence intervals for material samples; or providing a faster processing of sensor data. In particular, the present disclosure provides technical solutions to technical problems, including, but not limited to, hologram modification; image/signal blurring; application of combined blurring techniques; and/or subsequent image reconstruction, resulting in a faster, more thorough and/or more efficient processing of generated images and thus of material samples or other target compositions being imaged.

The embodiments disclosed herein thus provide improvements to material analysis technology (e.g., improvements in the computer technology supporting material analysis, among other improvements).

As used herein, the phrase "based on" should be understood to mean "based at least in part on," unless otherwise specified.

As u sed herein, the term "compound" can refer to a single material, multiple materials, composition, sample, solution, product, etc.

As used herein, the term "data" can comprise metadata.

As used herein, the terms "entity," "requesting entity," and "user entity" can refer to a machine, device, component, hardware, software, smart device, party, organization, individual and/or human.

One or more example embodiments are now described with reference to the drawings, where like referenced numerals are used to refer to like drawing elements throughout. In the following description, for purposes of explanation, numerous specific details are set forth in order to provide a more thorough understanding of the one or more example embodiments. It is evident in various cases, however, that the one or more example embodiments can be practiced without these specific details.

Further, it should be appreciated that the embodiments depicted in one or more figures described herein are for illustration only, and as such, the architecture of embodiments is not limited to the systems, devices and/or components depicted therein, nor to any particular order, connection and/or coupling of systems, devices and/or components depicted therein.

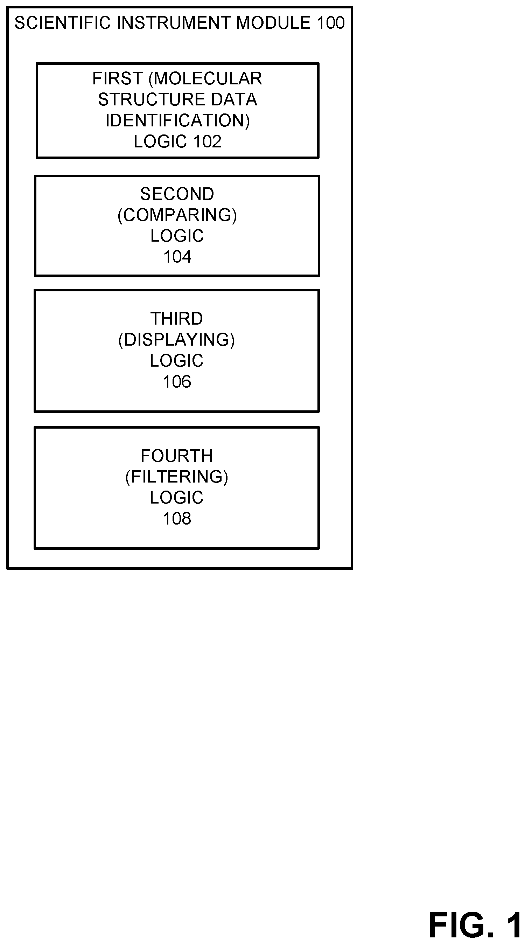

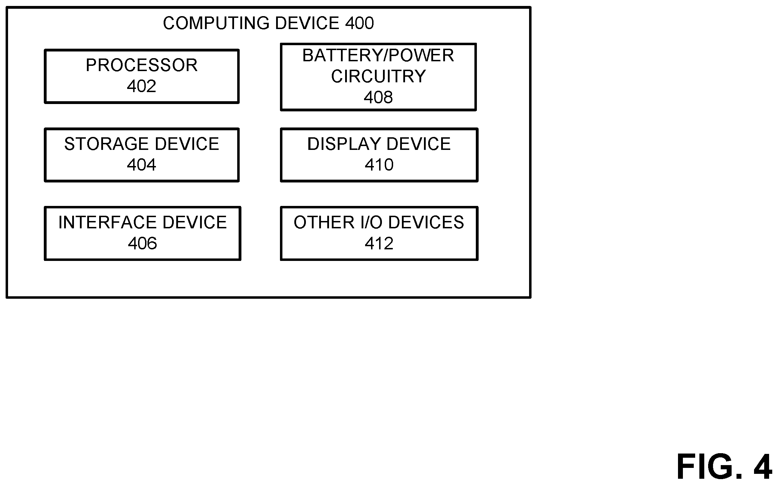

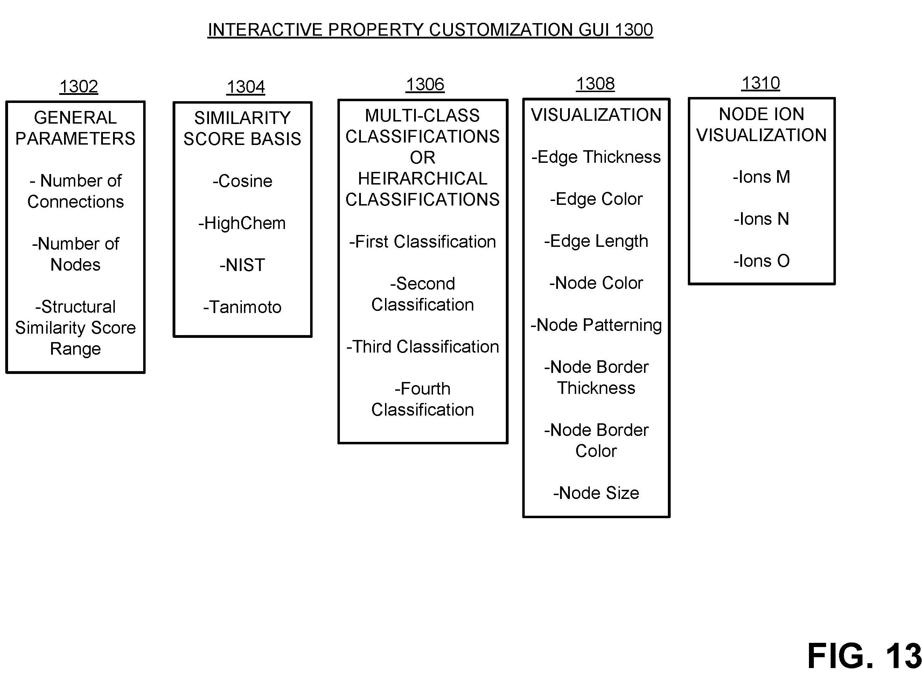

Turning now in particular to the one or more figures, and first to FIG. 1, illustrated is a block diagram of a scientific instrument module 100 for performing material analysis operations using a molecular network generation and/or visualization process, in accordance with various embodiments described herein. The scientific instrument module 100 can be implemented by circuitry (e.g., including electrical and/or optical components), such as a programmed computing device. The logic of the scientific instrument module 100 can be included in a single computing device or can be distributed across multiple computing devices that are in communication with each other as appropriate. Examples of computing devices that can, singly or in combination, implement the scientific instrument module 100 are discussed herein with reference to the computing device 400 of FIG. 4, and examples of systems of interconnected computing devices, in which the scientific instrument module 100 can be implemented across one or more of the computing devices, is discussed herein with reference to the scientific instrument system 1300 of FIG. 13.

The scientific instrument module 100 can include first logic 102, second logic 104, third logic 106, and fourth logic 108. As used herein, the term "logic" can include an apparatus that is to perform a set of operations associated with the logic. For example, any of the logic elements included in the module 100 can be implemented by one or more computing devices programmed with instructions to cause one or more processing devices of the computing devices to perform the associated set of operations. In a particular embodiment, a logic element can include one or more non-transitory computer-readable media having instructions thereon that, when executed by one or more processing devices of one or more computing devices, cause the one or more computing devices to perform the associated set of operations. As used herein, the term "module" can refer to a collection of one or more logic elements that, together, perform a function associated with the module. Different ones of the logic elements in a module can take the same form or can take different forms. For example, some logic in a module can be implemented by a programmed general-purpose processing device, while other logic in a module can be implemented by an application-specific integrated circuit (ASIC). In another example, different ones of the logic elements in a module can be associated with different sets of instructions executed by one or more processing devices. A module can omit one or more of the logic elements depicted in the associated drawing; for example, a module can include a subset of the logic elements depicted in the associated drawing when that module is to perform a subset of the operations discussed herein with reference to that module.

The first logic 102 can receive, find, locate, download, request, measure and/or otherwise determine data and/or metadata defining a set of molecular structures, such as from a user input and/or library datastore. That is, the first logic 102 can obtain data for being processed and for subsequent use in generating a molecular network cloud visual or updating a molecular structural library.

The second logic 104 can perform a comparing process by generally comparing molecular fingerprints representing and/or corresponding to the set of molecular structures to one another. In this way, one or more relationships can be determined, such as generating one or more structural similarity scores. That is, the second logic 104 can employ the output of the first logic 102 as a trigger for the second logic 104.

The third logic 106 can generate display data and further display, such as at a display device, a representative illustration corresponding to the comparison of the second logic 104. That is, the third logic 106 can employ an output of the second logic 104 to perform the third logic 106.

The fourth logic 108 can filter the molecular structure data employed by the third logic 106 to vary the representative illustration. That is, the fourth logic 108 can be employed by the third logic 106.

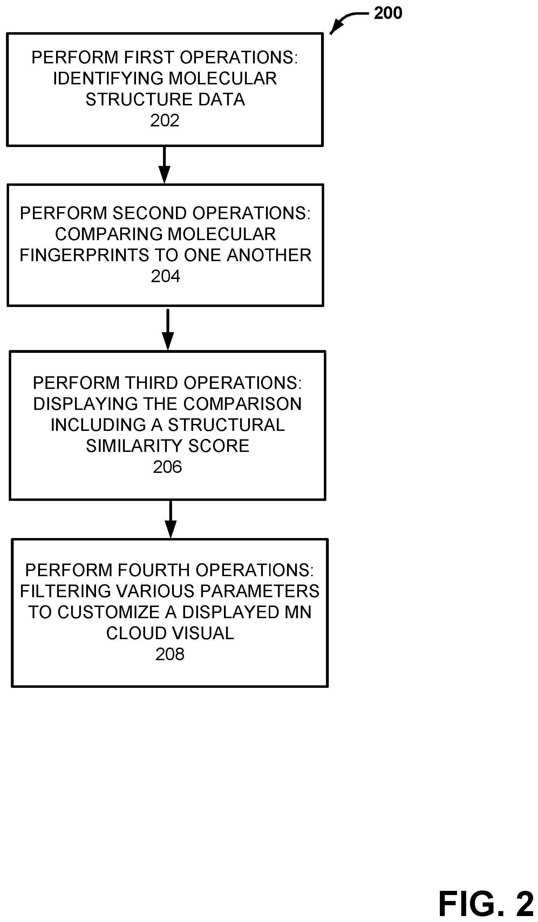

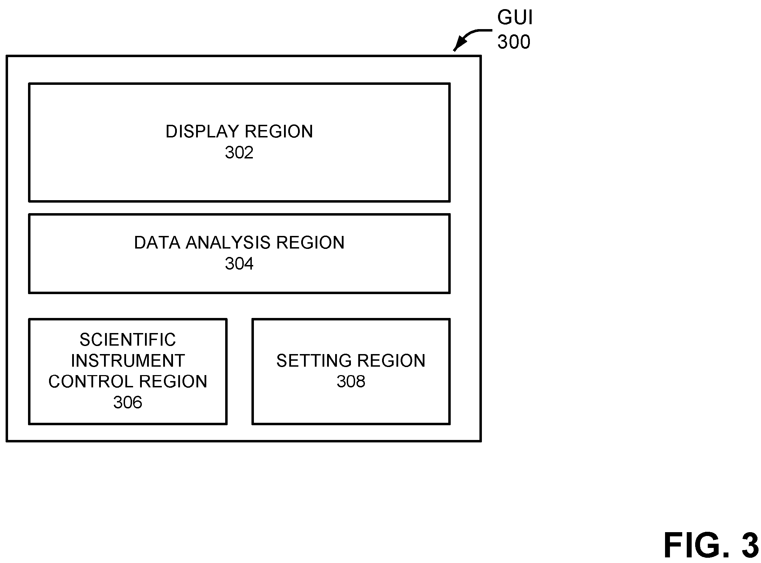

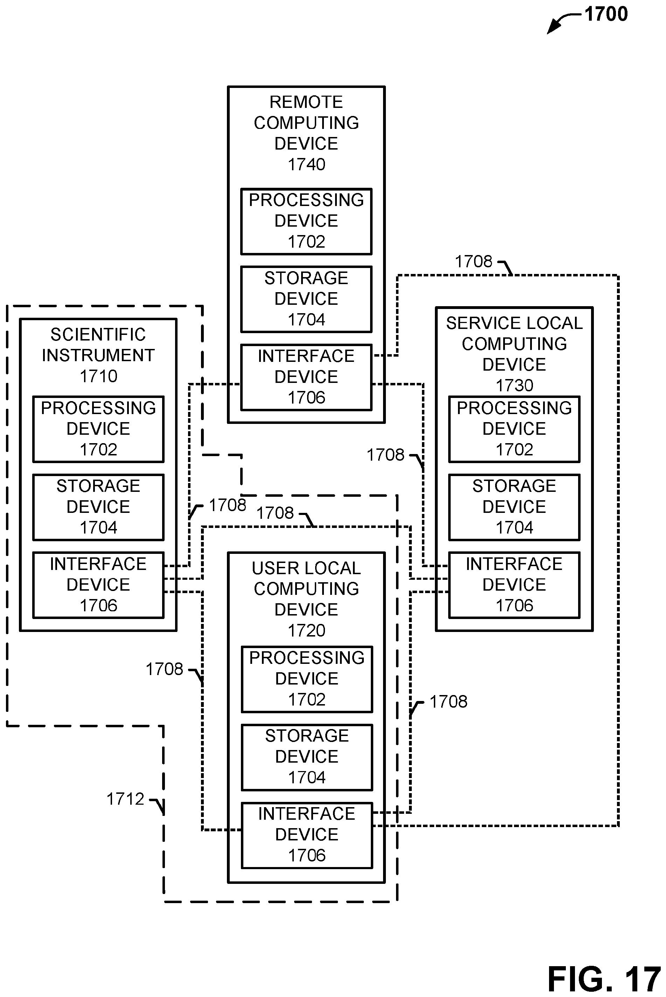

FIG. 2 illustrates a flow diagram of a method 200 of performing operations, by the scientific instrument module 100, in accordance with various embodiments. Although the operations of the method 200 can be illustrated with reference to particular embodiments disclosed herein (e.g., the scientific instrument module 100 discussed herein with reference to FIG. 1, the GUI 300 discussed herein with reference to FIG. 3, the computing device 400 discussed herein with reference to FIG. 4, and/or the scientific instrument system 1700 discussed herein with reference to FIG. 17), the method 200 can be used in any suitable setting to perform any suitable operations. Operations are illustrated once each and in a particular order in FIG. 2, but the operations can be reordered and/or repeated as desired and appropriate (e.g., different operations performed can be performed in parallel, as suitable).

At 202, first operations can be performed. For example, the first logic 102 of the module 100 can perform the first operations 202. The first operations 202 can include receiving, finding, locating, downloading, requesting, measuring and/or otherwise determining data and/or metadata defining a set of molecular structures.

At 204, second operations can be performed. For example, the second logic 104 of the module 100 can perform the second operations 204. The second operations 204 can comprise comparing molecular fingerprints to one another and generating at least a similarity score based on the comparing.

At 206, third operations can be performed. For example, the third logic 106 of the module 100 can perform the third operations 206. The third operations 206 can comprise generating display data based on the comparing and comprising data corresponding to the similarity score. The third operations 206 also can comprise employing the display data to visualize a MN cloud visual at a display device that illustrates the comparison, including the similarity score.

At 208, fourth operations can be performed. For example, the fourth logic 108 of the module 100 can perform the fourth operations 208. The fourth operations 208 can comprise filtering of underlying molecular structure data and/or comparison data employed by the third logic 106 and employed for the third operations 206. In this way, a modified visualization of a MN cloud visual can be generated and displayed by the third operations 206.

The scientific instrument methods disclosed herein can include interactions with a user entity (e.g., via the user local computing device 1720 discussed herein with reference to FIG. 17). These interactions can include providing information to the user entity (e.g., information regarding the operation of a scientific instrument such as the scientific instrument 1710 of FIG. 17, information regarding a sample being analyzed or other test or measurement performed by a scientific instrument, information retrieved from a local or remote database, or other information) or providing an option for a user entity to input commands (e.g., to control the operation of a scientific instrument such as the scientific instrument 1710 of FIG. 17, or to control the analysis of data generated by a scientific instrument), queries (e.g., to a local or remote database), or other information. In some embodiments, these interactions can be performed through a graphical user interface (GUI) that includes a visual display on a display device (e.g., the display device 410 discussed herein with reference to FIG. 4) that provides outputs to the user entity and/or prompts the user entity to provide inputs (e.g., via one or more input devices, such as a keyboard, mouse, trackpad, or touchscreen, included in the other I/O devices 412 discussed herein with reference to FIG. 4). The scientific instrument system 1700 disclosed herein can include any suitable GUIs for interaction with a user entity.

Turning next to FIG. 3, depicted is an example GUI 300 that can be used in the performance of one or more of the methods described herein, in accordance with various embodiments described herein. As noted above, the GUI 300 can be provided on a display device (e.g., the display device 410 discussed herein with reference to FIG. 4) of a computing device (e.g., the computing device 400 discussed herein with reference to FIG. 4) of a scientific instrument system (e.g., the scientific instrument system 1700 discussed herein with reference to FIG. 17), and a user entity can interact with the GUI 300 using any suitable input device (e.g., any of the input devices included in the other I/O devices 412 discussed herein with reference to FIG. 4) and input technique (e.g., movement of a cursor, motion capture, facial recognition, gesture detection, voice recognition, actuation of buttons, etc.).

The GUI 300 can include a data display region 302, a data analysis region 304, a scientific instrument control region 306, and a settings region 308. The particular number and arrangement of regions depicted in FIG. 3 is merely illustrative, and any number and arrangement of regions, including any desired features thereof, can be included in a GUI 300.

The data display region 302 can display data generated by a scientific instrument (e.g., the scientific instrument 1710 discussed herein with reference to FIG. 17). For example, the data display region 302 can display one or more output results which can comprise one or more fingerprints, one or more structural similarity scores, one or more cloud visuals and/or one or more cloud visual parameter control GUIs, without being limited thereto.

The data analysis region 304 can display the results of data analysis (e.g., the results of analyzing the data illustrated in the data display region 302 and/or other data). For example, the data analysis region 304 can display one or more of the output results of a filtering or comparison of molecular structure data. In one or more cases, the data analysis region 304 can display a list, flow chart or other schematic of the output results. In one or more example embodiments, the data display region 302 and the data analysis region 304 can be combined in the GUI 300 (e.g., to include data output from a scientific instrument, and some analysis of the data, in a common graph or region).

The scientific instrument control region 306 can include options that allow the user entity to control a scientific instrument (e.g., the scientific instrument 1710 discussed herein with reference to FIG. 17). For example, the scientific instrument control region 306 can include one or more controls for customizing a cloud visual, such as based on the GUI 1300 of FIG. 13, to be described below.

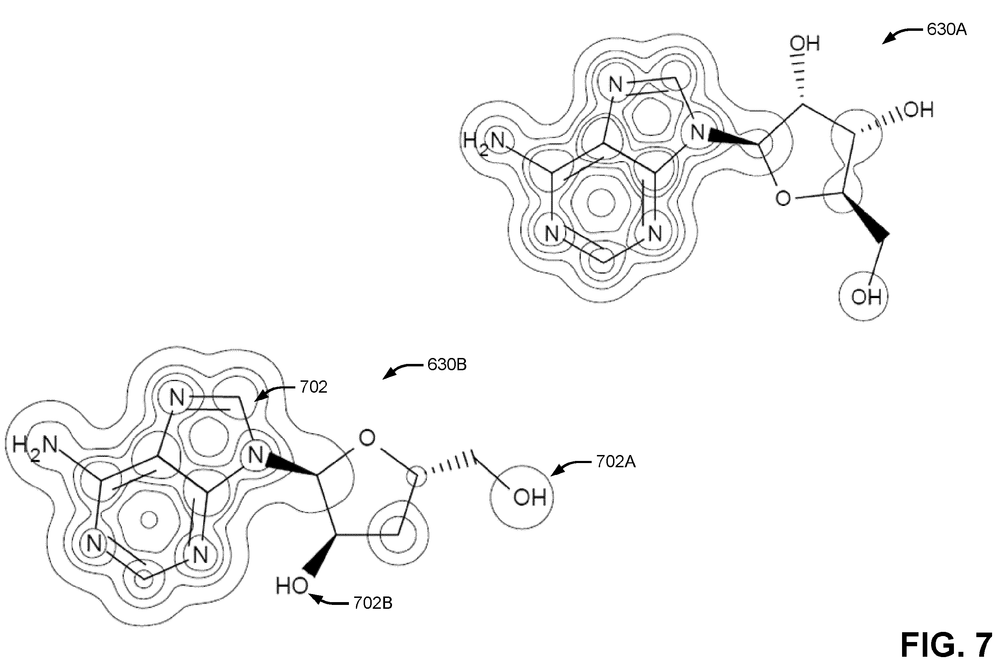

The settings region 308 can include options that allow the user entity to control the features and functions of the GUI 300 (and/or other GUIs) and/or perform common computing operations with respect to the data display region 302 and data analysis region 304 (e.g., saving data on a storage device, such as the storage device 404 discussed herein with reference to FIG. 4, sending data to another user entity, labeling data, etc.). For example, the settings region 308 can include one or more options to alter color, fill or format of illustrations, such as an illustration of any aspect of FIGS. 7-12 and/or other image, whether actual, representative and/or schematic, to be described below.

As noted above, the scientific instrument module 100 can be implemented by one or more computing devices. Accordingly, discussion next turns to FIG. 4, which illustrates a block diagram of a computing device 400 that can perform some or all of the scientific instrument methods disclosed herein, in accordance with various embodiments. In one or more example embodiments, the scientific instrument module 100 can be implemented by a single computing device 400 or by multiple computing devices 400. Further, as discussed below, a computing device 400 (or multiple computing devices 400) that implements the scientific instrument module 100 can be part of one or more of the scientific instrument 1710, the user local computing device 1720, the service local computing device 1730, or the remote computing device 1740 of FIG. 17.

The computing device 400 of FIG. 4 is illustrated as having a number of components, but any one or more of these components can be omitted or duplicated, as suitable for the application and setting. As illustrated, these components can include one or more of a processor 402, storage device 404, interface device 406, battery/power circuitry 408, display device 410 and other input/output (I/O) devices 412, as will be described below.

In one or more example embodiments, one or more of the components included in the computing device 400 can be attached to one or more motherboards and enclosed in a housing (e.g., including plastic, metal, and/or other materials). In one or more example embodiments, some these components can be fabricated onto a single system-on-a-chip (SoC) (e.g., an SoC can include one or more processors 402 and one or more storage devices 404). Additionally, in one or more example embodiments, the computing device 400 can omit one or more of the components illustrated in FIG. 4. In one or more example embodiments, the computing device 400 can include interface circuitry (not shown) for coupling to the one or more components using any suitable interface (e.g., a Universal Serial Bus (USB) interface, a High-Definition Multimedia Interface (HDMI) interface, a Controller Area Network (CAN) interface, a Serial Peripheral Interface (SPI) interface, an Ethernet interface, a wireless interface, or any other appropriate interface). For example, the computing device 400 can omit a display device 410, but can include display device interface circuitry (e.g., a connector and driver circuitry) to which a display device 410 can be coupled.

The computing device 400 can include the processor 402 (e.g., one or more processing devices). As used herein, the term "processing device" can refer to any device or portion of a device that processes electronic data from registers and/or memory to transform that electronic data into other electronic data that can be stored in registers and/or memory. The processor 402 can include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 400 can include a storage device 404 (e.g., one or more storage devices). The storage device 404 can include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In one or more example embodiments, the storage device 404 can include memory that shares a die with a processor 402. In such an embodiment, the memory can be used as cache memory and can include embedded dynamic random-access memory (eDRAM) or spin transfer torque magnetic random-access memory (STT-MRAM), for example. In one or more example embodiments, the storage device 404 can include non-transitory computer readable media having instructions thereon that, when executed by one or more processing devices (e.g., the processor 402), cause the computing device 400 to perform any appropriate ones of or portions of the methods disclosed herein.

The computing device 400 can include an interface device 406 (e.g., one or more interface devices 406). The interface device 406 can include one or more communication chips, connectors, and/or other hardware and software to govern communications between the computing device 400 and other computing devices. For example, the interface device 406 can include circuitry for managing wireless communications for the transfer of data to and from the computing device 400. The term "wireless" and its derivatives can be used to describe circuits, devices, systems, methods, techniques, communications channels, etc., that can communicate data through the use of modulated electromagnetic radiation through a nonsolid medium. The term does not imply that the associated devices do not contain any wires, although in one or more example embodiments the associated devices might not contain any wires. Circuitry included in the interface device 406 for managing wireless communications can implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In one or more example embodiments, circuitry included in the interface device 406 for managing wireless communications can operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In one or more example embodiments, circuitry included in the interface device 406 for managing wireless communications can operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In one or more example embodiments, circuitry included in the interface device 406 for managing wireless communications can operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In one or more example embodiments, the interface device 406 can include one or more antennas (e.g., one or more antenna arrays) to receipt and/or transmission of wireless communications.

In one or more example embodiments, the interface device 406 can include circuitry for managing wired communications, such as electrical, optical, or any other suitable communication protocols. For example, the interface device 406 can include circuitry to support communications in accordance with Ethernet technologies. In one or more example embodiments, the interface device 406 can support both wireless and wired communication, and/or can support multiple wired communication protocols and/or multiple wireless communication protocols. For example, a first set of circuitry of the interface device 406 can be dedicated to shorter-range wireless communications such as Wi-Fi or Bluetooth, and a second set of circuitry of the interface device 406 can be dedicated to longer-range wireless communications such as global positioning system (GPS), EDGE, GPRS, CDMA, WiMAX, LTE, EV-DO, or others. In one or more example embodiments, a first set of circuitry of the interface device 406 can be dedicated to wireless communications, and a second set of circuitry of the interface device 406 can be dedicated to wired communications.

The computing device 400 can include battery/power circuitry 408. The battery/power circuitry 408 can include one or more energy storage devices (e.g., batteries or capacitors) and/or circuitry for coupling components of the computing device 400 to an energy source separate from the computing device 400 (e.g., AC line power).

The computing device 400 can include a display device 410 (e.g., multiple display devices). The display device 410 can include any visual indicators, such as a heads-up display, a computer monitor, a projector, a touchscreen display, a liquid crystal display (LCD), a light-emitting diode display, or a flat panel display.

The computing device 400 can include other input/output (I/O) devices 412. The other I/O devices 412 can include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 400, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 400 can have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

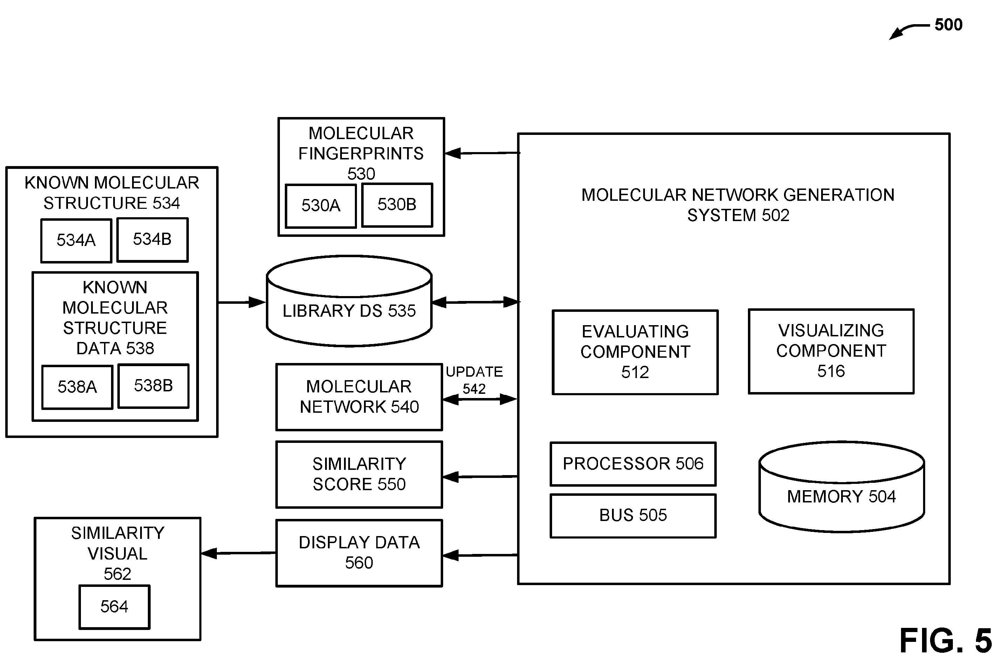

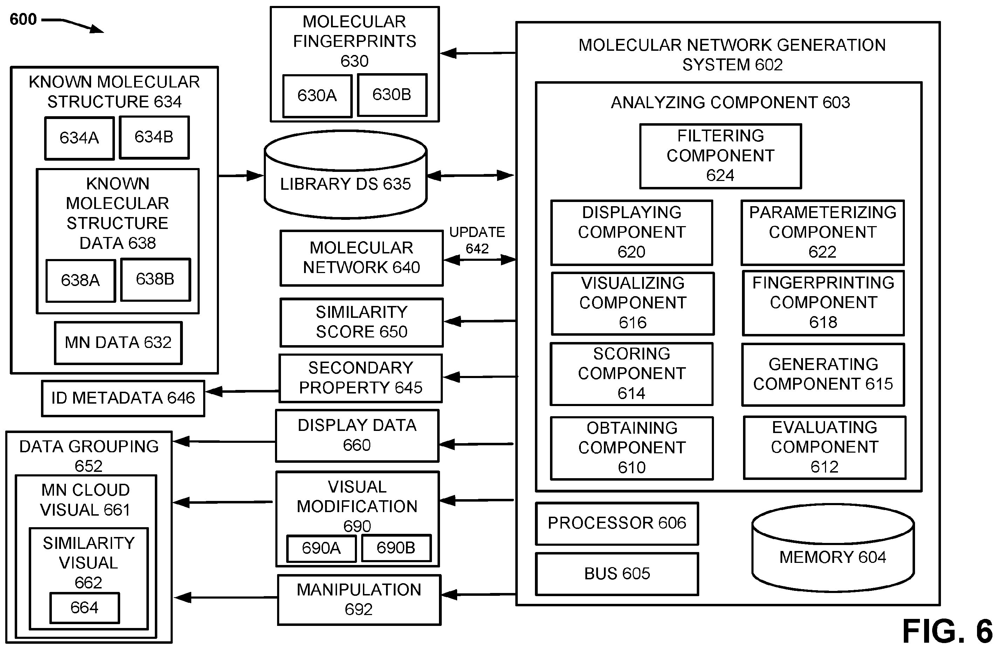

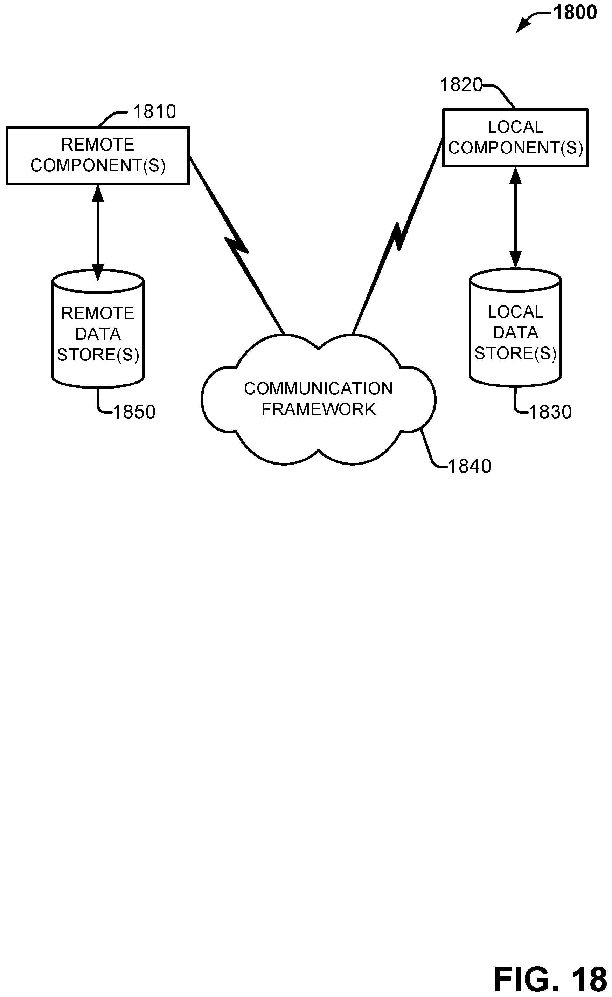

Referring now to FIGS. 5 and 6, in one or more example embodiments, the non-limiting systems 500 and/or 600 illustrated at FIGS. 5 and 6, and/or systems thereof, can further comprise one or more computer and/or computing-based elements described herein with reference to a computing environment, such as the computing environment 1800 illustrated at FIG. 18. In one or more described embodiments, computer and/or computing-based elements can be used in connection with implementing one or more of the systems, devices, components and/or computer-implemented operations shown and/or described in connection with FIGS. 5 and/or 6 and/or with other figures described herein.

Turning first to FIG. 5, the figure illustrates a block diagram of an example, non-limiting system 500 that can comprise a molecular network generation system 502 and a library datastore (DS) 535. The molecular network generation system 502 can generally facilitate generation of a molecular network 540 via updating of the molecular network 540 (e.g., via an update 542).

In one or more example embodiments, the molecular network generation system 502 can be at least partially comprised by the computing device 400.

It is noted that the molecular network generation system 502 is only briefly detailed to provide but a lead-in to a more complex and/or more expansive molecular network generation system 602 as illustrated at FIG. 6. That is, further detail regarding processes that can be performed by one or more example embodiments described herein will be provided below relative to the non-limiting system 600 of FIG. 6.

Still referring to FIG. 5, the molecular network generation system 502 can comprise at least a memory 504, bus 505, processor 506, evaluating component 512 and/or visualizing component 516. The processor 506 can be the same as the processor 402, comprised by the processor 402 or different therefrom. The memory 504 can be the same as the storage device 404, comprised by the storage device 404 or different therefrom.

Using the above-noted components, the molecular network generation system 502 can facilitate a process to at least partially generate the molecular network (MN) 540, such as by updating the library datastore 535, and thus the molecular network 540 that employs the library datastore 535, based on an update 542.

Generally, the evaluating component 512 can execute a comparison of a first molecular fingerprint 530A, comprising first molecular structure data 538A of a first molecular structure 534A of molecular structures 534, to a second molecular fingerprint 530B, comprising second molecular structure data 538B of a second molecular structure 534B of the molecular structures 534.

The evaluating component 512 further can generally determine whether there is another known molecular structure data 538 against which to compare the first molecular structure data 538A or the second molecular structure data 538B. More particularly, the evaluating component 512 can generally determine whether there is another known molecular structure data 538 for which to generate a molecular fingerprint 530 to be compared to the first molecular fingerprint 530A and/or the second molecular fingerprint 530B.

The visualizing component 516 generally can generate display data 560 for visualizing a similarity visual 562 illustrating representations of the first molecular structure 534A, the second molecular structure 534B, and a structural similarity score 550 resulting from the comparison.

As a result of these components, the data generated can be stored, such as at the library datastore 535, and thus the molecular network 540 can be generated and/or updated.

The evaluating component 512 and/or visualizing component 516 can be operatively coupled to the processor 506 which can be operatively coupled to the memory 504. The bus 505 can provide for the operative coupling. The processor 506 can facilitate execution of the evaluating component 512 and/or visualizing component 516. The evaluating component 512 and/or visualizing component 516 can be stored at the memory 504.

In general, the non-limiting system 500 can employ any suitable method of communication (e.g., electronic, communicative, internet, infrared, fiber, etc.) to provide communication between the molecular network generation system 502 and/or any device associated with a user entity.

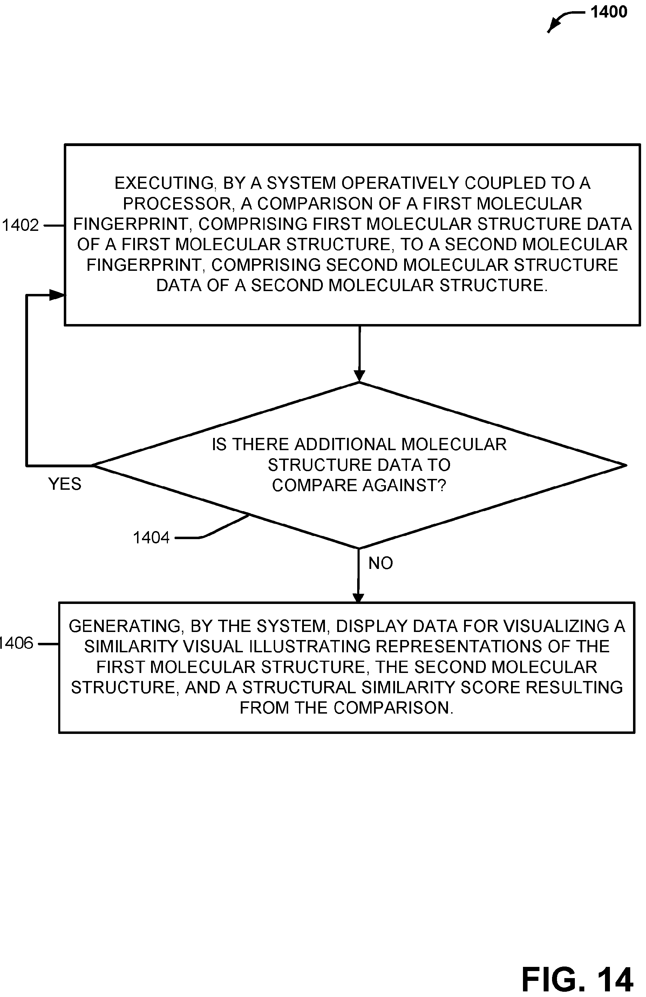

As a summary of the above-described components and functions thereof, referring next only briefly to FIG. 14, illustrated is a flow diagram of an example, non-limiting method 1400 that can facilitate a process to generate and/or update a MN, in accordance with one or more example embodiments described herein, such as the non-limiting system 500 of FIG. 5. While the non-limiting method 1400 is described relative to the non-limiting system 500 of FIG. 5, the non-limiting method 1400 can be applicable also to other systems described herein, such as the non-limiting system 600 of FIG. 6. Repetitive description of like elements and/or processes employed in respective embodiments is omitted for sake of brevity.

At 1402, the non-limiting method 1400 can comprise executing, by a system (e.g., evaluating component 512) coupled to a processor (e.g., processor 506), a comparison of a first molecular fingerprint (e.g., first molecular fingerprint 530A), comprising first molecular structure data (e.g., first molecular structure data 538A) of a first molecular structure (e.g., first molecular structure 534A), to a second molecular fingerprint (e.g., second molecular fingerprint 530B), comprising second molecular structure data (e.g., second molecular structure data 538B) of a second molecular structure (e.g., second molecular structure 534B).

At 1404, the non-limiting method 1400 can comprise determining, by the system (e.g., evaluating component 512), whether there is another known molecular structure data (e.g., known molecular structure data 538) against which to compare the first molecular structure data or the second molecular structure data. If yes, the non-limiting method 1400 can proceed back to step 1402. If not, the non-limiting method can proceed forward to step 1406.

At 1406, the non-limiting method 1400 can comprise generating, by the system (e.g., visualizing component 516) display data (e.g., display data 560) for visualizing a similarity visual (e.g., similarity visual 562) illustrating representations (e.g., representations 564) of the first molecular structure, the second molecular structure, and a structural similarity score (e.g., structural similarity score 650) resulting from the comparison.

Turning next to FIG. 6, a non-limiting system 600 is illustrated that can comprise a molecular network generation system 602 and a library datastore (DS) 635. Repetitive description of like elements and/or processes employed in respective embodiments is omitted for sake of brevity. Description relative to an embodiment of FIG. 5 can be applicable to an embodiment of FIG. 6. Likewise, description relative to an embodiment of FIG. 6 can be applicable to an embodiment of FIG. 5.

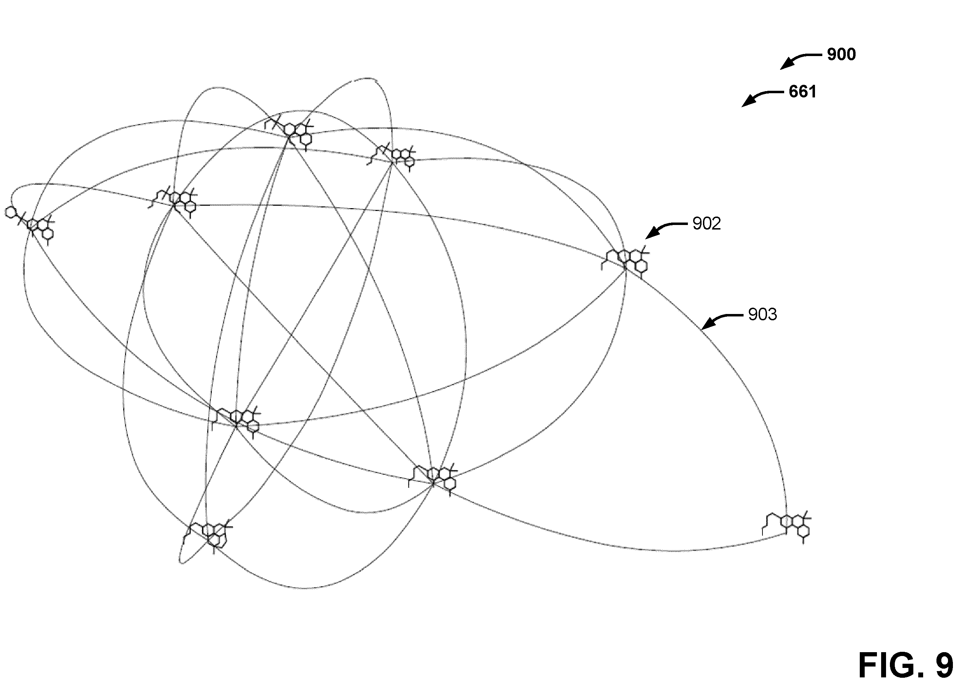

Generally, the molecular network generation system 602 can facilitate a process to at least partially generate the molecular network (MN) 640, such as by updating the library datastore 635, and thus the molecular network 640 that employs the library datastore 635, based on an update 642. In one or more example embodiments, the MN generation system 602 can facilitate a process to generate and/or display a MN cloud visual 661 (see, e.g., FIG. 9, to be discussed below).

In one or more example embodiments, the molecular network generation system 602 can be at least partially comprised by the computing device 400.

One or more communications between one or more components of the non-limiting system 600 can be provided by wired and/or wireless means including, but not limited to, employing a cellular network, a wide area network (WAN) (e.g., the Internet), and/or a local area network (LAN). Suitable wired or wireless technologies for supporting the communications can include, without being limited to, wireless fidelity (Wi-Fi), global system for mobile communications (GSM), universal mobile telecommunications system (UMTS), worldwide interoperability for microwave access (WiMAX), enhanced general packet radio service (enhanced GPRS), third generation partnership project (3GPP) long term evolution (LTE), third generation partnership project 2 (3GPP2) ultra-mobile broadband (UMB), high speed packet access (HSPA), Zigbee and other 802.XX wireless technologies and/or legacy telecommunication technologies, BLUETOOTH®, Session Initiation Protocol (SIP), ZIGBEE®, RF4CE protocol, WirelessHART protocol, 6LoWPAN (Ipv6 over Low power Wireless Area Networks), Z-Wave, an advanced and/or adaptive network technology (ANT), an ultra-wideband (UWB) standard protocol and/or other proprietary and/or non-proprietary communication protocols.

The molecular network generation system 602 can be associated with, such as accessible via, a cloud computing environment, such as the cloud computing environment 1800 of FIG. 18.

The molecular network generation system 602 can comprise a plurality of components. The components can comprise a memory 604, processor 606, bus 605, obtaining component 610, evaluating component 612, scoring component 614, generating component 615, visualizing component 616, fingerprinting component 618, displaying component 620, parameterizing component 622, and/or filtering component 624. Using these components, the molecular network generation system 602 can update the molecular network 640, generate a MN cloud visual 661 and/or provide a visual modification 690 and/or manipulation 692 of the MN cloud visual 661.

Discussion next turns to the processor 606, memory 604 and bus 605 of the molecular network generation system 602. For example, in one or more example embodiments, the molecular network generation system 602 can comprise the processor 606 (e.g., computer processing unit, microprocessor, classical processor, quantum processor and/or like processor). In one or more example embodiments, a component associated with molecular network generation system 602, as described herein with or without reference to the one or more figures of the one or more example embodiments, can comprise one or more computer and/or machine readable, writable and/or executable components and/or instructions that can be executed by processor 606 to provide performance of one or more processes defined by such component and/or instruction. In one or more example embodiments, the processor 606 can comprise the obtaining component 610, evaluating component 612, scoring component 614, generating component 615, visualizing component 616, fingerprinting component 618, displaying component 620, parameterizing component 622, and/or filtering component 624.

In one or more example embodiments, the molecular network generation system 602 can comprise the computer-readable memory 604 that can be operably connected to the processor 606. The memory 604 can store computer-executable instructions that, upon execution by the processor 606, can cause the processor 606 and/or one or more other components of the molecular network generation system 602 (e.g., obtaining component 610, evaluating component 612, scoring component 614, generating component 615, visualizing component 616, fingerprinting component 618, displaying component 620, parameterizing component 622, and/or filtering component 624) to perform one or more actions. In one or more example embodiments, the memory 604 can store computer-executable components (e.g., obtaining component 610, evaluating component 612, scoring component 614, generating component 615, visualizing component 616, fingerprinting component 618, displaying component 620, parameterizing component 622, and/or filtering component 624).

The molecular network generation system 602 and/or a component thereof as described herein, can be communicatively, electrically, operatively, optically and/or otherwise coupled to one another via a bus 605. Bus 605 can comprise one or more of a memory bus, memory controller, peripheral bus, external bus, local bus, quantum bus and/or another type of bus that can employ one or more bus architectures. One or more of these examples of bus 605 can be employed.

In one or more example embodiments, the molecular network generation system 602 can be coupled (e.g., communicatively, electrically, operatively, optically and/or like function) to one or more external systems (e.g., a non-illustrated electrical output production system, one or more output targets and/or an output target controller), sources and/or devices (e.g., classical and/or quantum computing devices, communication devices and/or like devices), such as via a network. In one or more example embodiments, one or more of the components of the molecular network generation system 602 and/or of the non-limiting system 600 can reside in the cloud, and/or can reside locally in a local computing environment (e.g., at a specified location).

In addition to the processor 606 and/or memory 604 described above, the molecular network generation system 602 can comprise one or more computer and/or machine readable, writable and/or executable components and/or instructions that, when executed by processor 606, can provide performance of one or more operations defined by such component and/or instruction.

Discussion next turns to the additional components of the molecular network generation system 602 (e.g., obtaining component 610, evaluating component 612, scoring component 614, generating component 615, visualizing component 616, fingerprinting component 618, displaying component 620, parameterizing component 622, and/or filtering component 624), generally, the molecular network generation system 602 can perform a set of processes that can be separated into various steps comprising, but not limited to: generating of underlying molecular network (MN) data 632, updating and/or generating a molecular network 640, generation of a MN cloud visual 661, and/or manipulation of the MN 640.

First, it is noted that in one or more example embodiments, the obtaining component 610, evaluating component 612, scoring component 614, generating component 615, visualizing component 616, fingerprinting component 618, displaying component 620, parameterizing component 622, and/or filtering component 624 can be implemented independently, without one or more other of the obtaining component 610, evaluating component 612, scoring component 614, generating component 615, visualizing component 616, fingerprinting component 618, displaying component 620, parameterizing component 622, and/or filtering component 624. Additionally and/or alternatively, the obtaining component 610, evaluating component 612, scoring component 614, generating component 615, visualizing component 616, fingerprinting component 618, displaying component 620, parameterizing component 622, and/or filtering component 624 can be comprised by a high-level analyzing component 603, one or more of the below-described functions of the obtaining component 610, evaluating component 612, scoring component 614, generating component 615, visualizing component 616, fingerprinting component 618, displaying component 620, parameterizing component 622, and/or filtering component 624 can be performed by the high-level analyzing component 603, and/or the obtaining component 610, evaluating component 612, scoring component 614, generating component 615, visualizing component 616, fingerprinting component 618, displaying component 620, parameterizing component 622, and/or filtering component 624 can be omitted with the high-level analyzing component 603 performing one or more of the below-described functions of the one or more omitted obtaining component 610, evaluating component 612, scoring component 614, generating component 615, visualizing component 616, fingerprinting component 618, displaying component 620, parameterizing component 622, and/or filtering component 624.

Turning first to the obtaining component 610, this component can generally acquire (e.g., obtain, locate, identify, request, download, etc.) molecular structure data 638 for processing. The molecular structure data 638 can correspond to and/or define one or more known molecular structures 634. In one or more example embodiments, the obtaining component 610 can intercept, read and/or copy a query signal, communication, etc. intended for the molecular network 640 (e.g., where the MN 640 employs a processor, such as the processor 606 or another processor). The molecular structure data 638 can be in any suitable form, comprise data and/or metadata, can be based on and/or comprised by a spectrum or data underlying a spectrum, etc.

Turning briefly to FIG. 7, generally the fingerprinting component 618 can generate a set of molecular fingerprints 630. In one or more cases, this generation can define a molecular structure 634, based on its molecular structure data 638, as comprising different fingerprint bits 702. For example, fingerprint bits 702 can comprise types of ions, atoms, bonds, etc., such as a hydroxide (OH) molecule or a carbon ring, such as an oxetane, or other four-membered heterocyclic ring with an oxygen molecule, as illustrated at fingerprints 630A and 630B of FIG. 2.

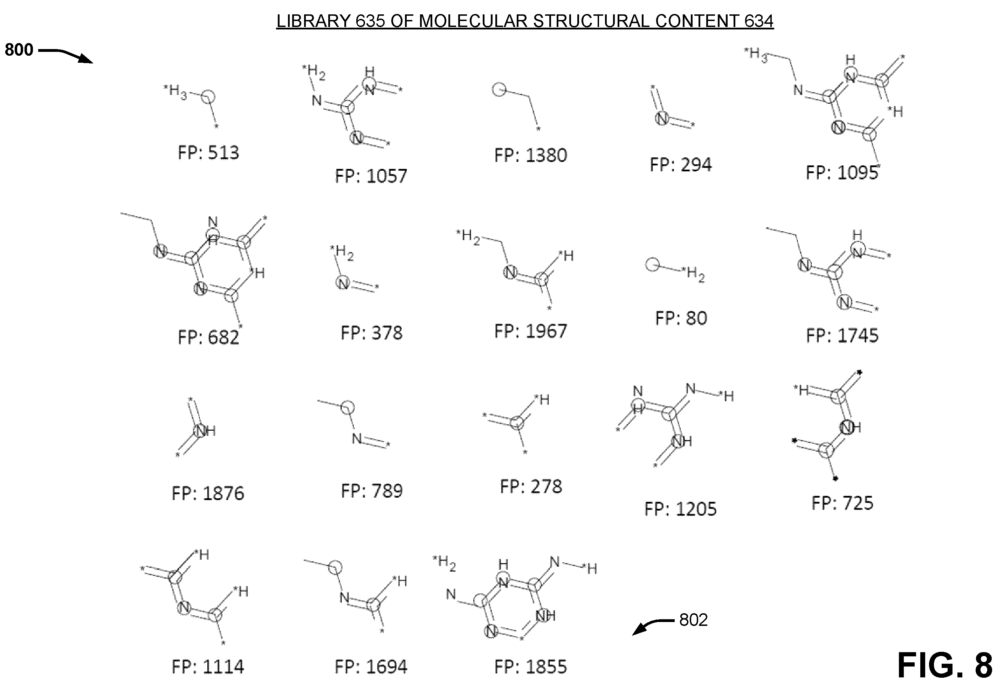

For example, the fingerprinting component 618 can generate a first molecular fingerprint 630A based on a first molecular structure 634A, wherein the first molecular fingerprint 630A is unique to the first molecular structure 634A and has identification metadata 802, that is unique to the first molecular fingerprint 630A, associated therewith. See, for example, FIG. 8, providing a schematic illustration 800 of a library 635 of molecular structural content (e.g., comprising molecular structures 634). More particularly, the schematic illustration 800 illustrates a set of molecular fingerprints (FPs) 630, illustrated based on fingerprinting by the fingerprinting component 618 and each comprising a unique identification label based on respective identification metadata 802. For example, an identification label can state FP:513 indicating the icon as a fingerprint (FP) having a numerical ID (e.g., 513).

In one or more example embodiments, an open-source fingerprinting application can be employed to define molecular structures 634 in terms of visual fingerprints 630.

Based on the fingerprinting, the evaluating component 612 can generally generate pair-wise structural similarities for each pair of fingerprints 630 of a set, such as of a whole of the library datastore 635. The evaluating component 612 can likewise generate MN data 643 defining the comparisons and/or generate a data element to store the MN data 632, such as a similarity matrix or other data element. The MN data 632 can be in any suitable form.

For example, relative to the molecular fingerprints 630A, 630B of FIGS. 6 and 7, the evaluating component 612 can execute a comparison of the first molecular fingerprint 630A, comprising the first molecular structure data 638A of the first molecular structure 634A, to a second molecular fingerprint 630B, comprising second molecular structure data 638B of a second molecular structure 634B. Data resulting from the comparison (e.g., generated by the evaluating component 612), can be stored as MN data 632 at the library datastore 635, for example.

Based on the comparing, the scoring component 614 can generally generate a structural similarity score 650 based on comparing the fingerprints 630. In one or more example embodiments, one or more of the similarity scores 650 can be generated based on the output of the evaluating component 612. In one or more example embodiments, generating of one or more similarity scores 650 can comprise executing one or more sub-comparisons of common and/or uncommon fingerprint bits 702 between pairs of fingerprints 630 (e.g., for pair-wise similarity). For example, relative to the molecular fingerprints 630A, 630B of FIGS. 6 and 7, this can comprise executing one or more sub-comparisons of first fingerprint bits 702A that are common to each of the first molecular fingerprint 630A and the second molecular fingerprint 630B, and of second fingerprint bits 702B that are uncommon, to both of the first molecular fingerprint 630A and the second molecular fingerprint 630B.

Indeed, the scoring component 614 can perform such generation for each pairing of the molecular structures 634 (e.g., pairings of the molecular structure data 638 for the respective pairings of the known molecular structures 634) with each of the plurality of known molecular structure data 638 employed by the evaluating component 612, based on each respective comparison output thereof.

A structural similarity score 650 can thus describe a similarity between different aspects (e.g., ions, bonds, etc.) of the molecular fingerprints 630 corresponding to the molecular structure data 638.

In one or more example embodiments, the scoring component 614 can employ a score algorithm, program, code and/or application such as a cosine, Tanimoto, Euclid, Dice, HighChem-HighRes, and/or National Institute of Standards and Technologies (NIST)-based algorithm.

In one or more example cases, a structural similarity score 650, such as based on Tanimoto similarity, can be based on a scale of 0 to 1 inclusive, with 0 meaning no similarity between the compared molecular fingerprints 630 or molecular structure data 638 and 1 meaning exact similarity between the compared molecular fingerprints 630 or molecular structure data 638. Any fragmentation, subdivisions, etc. between 0 and 1 can be employed, e.g., via any suitable number of decimal places.

In one or more example embodiments, a Tanimoto similarity score can be employed based on Tanimoto coefficients. A Tanimoto coefficient can be a ratio of features common to both molecules to the total number of features. For example, the Equation 1: $\frac{c}{a+b+c}$ can be employed, where for this Equation 1, a is the count of bits that are in object A but that are not in object B, b is the count of bits that are in object B but that are not in object A, and c is the count of bits that are in both object A and object B. Put another way, this can be described as

$$\frac{A \text{ intersect } B}{A+B-(A \text{ intersect } B)}.$$

In one or more other example embodiments, a Euclid similarity score can be employed based on Equation 2. For example, Equation 2: $\sqrt{\frac{c+d}{a+b+c+d}}$ can be employed, where for this Equation 1, a is the count of bits that are in object A but that are not in object B, b is the count of bits that are in object B but that are not in object A, c is the count of bits that are in both object A and object B, and d is the count of bits that are in neither object A nor object B.

In one or more other example embodiments, a cosine similarity score can be employed based on Equation 3. For example, Equation 3: $\frac{c}{\sqrt{(a+c)*(b+c)}}$ can be employed, where for this Equation 1, a is the count of bits that are in object A but that are not in object B, b is the count of bits that are in object B but that are not in object A, and c is the count of bits that are in both object A and object B

In one or more other example embodiments, a Dice similarity score can be employed based on Equation 4. For example, Equation 4: $\frac{2*c}{(a+c)+(b+c)}$ can be employed, where for this Equation 1, a is the count of bits that are in object A but that are not in object B, b is the count of bits that are in object B but that are not in object A, and c is the count of bits that are in both object A and object B.

Discussion turns again to the evaluating component 612. Based on output of the scoring component 614, one or more organization and/or storage operations can be performed. In one or more example embodiments, an open-source pair-wise structural similarity application can be employed to provide the comparisons and/or data element, and thus to assist the evaluating component 612.

In one or more example embodiments, the evaluating component 612 further can generally determine whether there is another known molecular structure data 638 against which to compare the first molecular structure data 638A or the second molecular structure data 638B, such as of a selected or filtered group, or of the whole library datastore 635. More particularly, the evaluating component 612 can generally determine whether there is another known molecular structure data 638 for which to generate a molecular fingerprint 630 (e.g., by the fingerprinting component 618) to be compared to the first molecular fingerprint 630A and/or the second molecular fingerprint 630B.

Additionally, and/or alternatively, the evaluating component 612 can further associate a secondary property 645 of one molecular structure data with another molecular structure data, based on the comparison by the evaluating component 612 of the first molecular structure data 638A and the second molecular structure data 638B.

That is, the evaluating component 612 can identify identification metadata 646 (e.g., ID metadata 646) associated with the molecular structure data 638, which metadata 646 can define a secondary property 645. Such secondary property metadata 646 can be stored with the molecular structure data 638 and/or separate therefrom. In one or more embodiments, where the molecular structure data 638 is associated with a query to the molecular network 640, secondary property metadata 646 can be comprised by and/or separate from the molecular structure data 638.

This associating, with the comparing of pairs of the molecular structure data 638, and as with the generating of respective similarity scores 650, can be performed for a plurality of different molecular structure data pairs, such as for each combination of a molecular structure data associated with a query and each molecular structure data 638 comprised by and/or employed by a molecular network 640 (e.g., comprised by a library datastore 635).

A secondary property 645 can be based on and/or comprise any one or more of the properties provided at FIG. 9, and/or any one or more properties provided above and/or below, without being limited thereto. For example, a secondary property 645 can be based on and/or comprise one or more physical properties, chemical properties, compound classes, fragmentation kinetics, collision energies, chemical formulas, neutral losses, pick counts, commercial applications and/or industrial applications. In one or more embodiments, any one or more such properties can be provided by the system as one that is associated with a parameter for multi-class or hierarchical classification.

In one or more examples, a first secondary property 645 corresponding to the first molecular structure data 638A can be associated by the evaluating component 612 with the second molecular structure data 638B, based on a similarity score 650 defining a similarity level between the first molecular structure data 638A and the second molecular structure data 638B. Additionally, and/or alternatively, a second secondary property 645 corresponding to the second molecular structure data 638B can be associated by the evaluating component 612 with the first molecular structure data 638A, based on a similarity score 650 defining a similarity level between the first molecular structure data 638A and the second molecular structure data 638B.

For example, based on a similarity score 650 satisfying (e.g., meeting and/or exceeding) a score threshold, the evaluating component 612 can determine to associate a secondary property 645 of one molecular structure data of the respective pair of molecular structure data corresponding to the similarity score 650 also with the other molecular structure data of the pair of molecular structure data.

Additionally, and/or alternatively, in one or more examples, a first secondary property 645 corresponding to first molecular structure data 638A can be associated by the evaluating component 612 with a second molecular structure data 638B, based on a plurality of similarity scores 650 defining respective similarity levels between the first molecular structure data 638A and a plurality of second molecular structure data (e.g., including the second molecular structure data 638B, such as from a library datastore 635 employed by a molecular network 640). Additionally, and/or alternatively, a second secondary property 645 corresponding to the second molecular structure data 638B can be associated by the evaluating component 612 with the first molecular structure data 638A, based on plurality of similarity scores 650 defining respective similarity levels between the first molecular structure data 638A and a plurality of second molecular structure data (e.g., including the second molecular structure data 638B, such as from a library datastore 635 employed by a molecular network 640).

For example, based on a quantity of the plurality of similarity scores 650 satisfying (e.g., meeting and/or exceeding) a score threshold, based on an aggregation of the quantity of similarity scores 650 satisfying a score threshold, or based on an aggregation of all similarity scores 650 associated with the first molecular structure data 638A satisfying a score threshold, the evaluating component 612 can determine to associate a secondary property 645 of one molecular structure data of the respective pair of molecular structure data corresponding to at least one similarity score 650 also with the other molecular structure data of the pair of molecular structure data. Additional associations can also be performed based thereon.

Additionally, and/or alternatively, in one or more examples, a first secondary property 645 corresponding to first molecular structure data 638A can be associated by the evaluating component 612 with a second molecular structure data 638B, based on there being at least a specified quantity of molecular structure data (e.g., known or unknown, such as known molecular structure data 638, such as from a library datastore 635 employed by a molecular network 640), including the second molecular structure data 638B, and satisfying a comparison criteria. Additionally, and/or alternatively, a second secondary property 645 corresponding to second molecular structure data 638B can be associated by the evaluating component 612 with the first molecular structure data 638A, based on there being at least a specified quantity of molecular structure data (e.g., known or unknown, such as from a library datastore 635 employed by a molecular network 640), including the second molecular structure data 638B satisfying a comparison criteria.

For example, at least a first quantity of second molecular structure data (e.g., a plurality of known molecular structure data 638) can each have a second secondary property 645 associated therewith. The first quantity of second molecular structure data can be those having been compared to the first molecular structure data 638A resulting in a validated similarity by the evaluating component 612, such as having at least a specified similarity score 650 (e.g., satisfying a threshold each and/or in aggregate, such as based on an aggregation of the respective similarity scores 650). Based on the first quantity being reached and/or exceeded (e.g., the quantity being a threshold to be satisfied as described herein), the second secondary property 645 can be associated with the first molecular structure data 638A, or the first secondary property 645 can be associated with the second molecular structure data, by the evaluating component 612.

Additionally, and/or alternatively, in one or more embodiments, a first secondary property 645 can only be associated with a second molecular structure data where the first molecular structure data 638A has a high priority associated therewith (e.g., satisfying a priority threshold), such as based on respective identification metadata 646 corresponding thereto.

Additionally, and/or alternatively, in one or more embodiments, one or more secondary properties 645 can be associated prior to or instead of one or more other secondary properties 645. Such determination can be made by the system, such as based on historical data obtained from the data store 635 and/or by selection by a user entity using a computer device communicatively couplable to the non-limiting system 600. In a case where a first selection is not comprised by metadata 646 corresponding to a molecular structure data, a second selection can next take priority.

Any two or more of the above-noted examples can be performed for a same molecular structure data. Any two or more such associations can be performed at least partially in parallel with one another.

Turning next to the generating component 615, this component can generally generate a grouping of molecular structure data 632 (such as a molecular structure data grouping 652) comprising the first molecular structure data 638A and the second molecular structure data 638B based on the similarity score 650 and on the associating of one or more secondary properties 645. A molecular structure data grouping 652 can comprise a list, matrix, log or any other grouping of data, metadata and/or labels defining a set of molecular structure data (e.g., any combination of known and/or unknown molecular structure data) that can be determined by the generating component 615 as being related, and thus having a relationship. The molecular structure data grouping 652 can be provided to a user entity (e.g., transmitted to and/or made available to a user entity computer device) in any suitable form, such as a list, matrix, log, etc. In one or more cases, the molecular structure data grouping 652 additionally and/or alternatively can be employed by the visualizing component 616 to generate a molecular network cloud visual 661, as described below.

The relationship can be generally based on a combination of similarity scores 650 and secondary properties 645 associated with the molecular structure data to be comprised by the molecular structure data grouping 652. In one or more examples, a molecular structure data grouping 652 can be based on a first specified range of similarity scores 650 and on a second specified range of one or more secondary properties 645. In one or more cases, the second specified range can be based on the first specified range, or vice versa.

In one or more cases, selection of the first specified range and/or the second specified range can be based on a determination by the generating component 615 and/or on data associated with query to the molecular network 640. Additionally, and/or alternatively, in one or more cases, selection of the first specified range can be based on a highest similarity score 650 associated with the first molecular structure data 638A (e.g., which first molecular structure data 638A can correspond to the query). Additionally, and/or alternatively, in one or more cases, selection of the second specified range can be based on a secondary property 645 having been associated by the evaluating component 612 (either associated to the first molecular structure data 638A or to the second molecular structure data 638B).

Next, the visualizing component 616 generally can generate display data 660 for visualizing a similarity visual 662 illustrating representations of the first molecular structure 634A, the second molecular structure 634B, and a structural similarity score 650 resulting from the comparison.

In one or more example embodiments, the visualizing component 616 generally can apply an update 642 to the molecular structural library (e.g., library datastore 635) based on the similarity scores 650 and data thereof. In one or more example embodiments, the visualizing component 616 can additionally and/or alternatively apply the update 642 to the MN 640 and/or direct an MN 640 to update based on the library datastore 635.

As a result of these components, the data generated can be stored, such as at the library datastore 635, and thus the molecular network 640 can be generated and/or updated.

Put another way, through use of the scoring component 614, and subsequent updating of the library datastore 635 underlying the MN 640, and thus by inherency updating the MN 640, poor relationship representation that is a deficiency of existing systems can be at least partially and/or fully remedied, thereby resulting in increased accuracy, efficiency and/or speed when visually illustrating the MN 640.

Discussion next turns to the generation of display data 660 by the visualizing component 616 and subsequent generation of a visualization (e.g., MN cloud visual 661 comprising one or more similarity visuals 662 based on one or more representations 664) based on the display data 660 by the displaying component 620.

Turning to the illustration 900 of FIG. 9, and still referring to FIG. 6, illustrated is an example MN cloud visual 661 of a portion of the MN 640, based on a plurality of similarity score 650 generations. Display data 660 underlying the MN cloud visual 661 can be generated by the visualizing component 616 and the display data 660 can be employed by the displaying component 620 to generate the MN cloud visual 661, such as at a display, such as the data display region 302 of the GUI 300 and/or display device 400. Generally, any suitable GUI, display, etc. communicatively couplable to the MN generation system 602 and/or non-limiting system 600 more generally can be employed.

The displaying component 620, based on this generation, can generate visual data for generating a visualization of the MN 640 (e.g., or a portion thereof). This can include generating visualization data to represent the various molecular structures 634 as nodes 902, and the structural similarity scores 650 as edges 903 extending between the respective nodes 902. The edge 903 can comprise text next to, adjacent to, contiguous therewith, etc. that includes numbers of the structural similarity score 650, for easy visual reference by a user entity.

For example, at the illustration 900 of FIG. 9, illustrated is a MN cloud visual 661, comprising representation, in a cloud format, of a plurality of relationships between molecular structures 634 (represented as the nodes 902), where the relationships are represented as edges 903 extending between the nodes 902. In one or more example embodiments, these edges 903 can represent the similarity scores 650. In one or more example embodiments, these edges can have text associated therewith listing the similarity scores 650. In one or more example embodiments, therefore, a single edge 903 can be employed between each pair of nodes 902 representing the respective pairwise similarity generated as described above. As noted, display data 660 underlying the nodes 902 and edges 903 can be generated by the visualizing component 616 and the display data 660 can be employed by the displaying component 620 to generate the nodes 902 and edges 903.

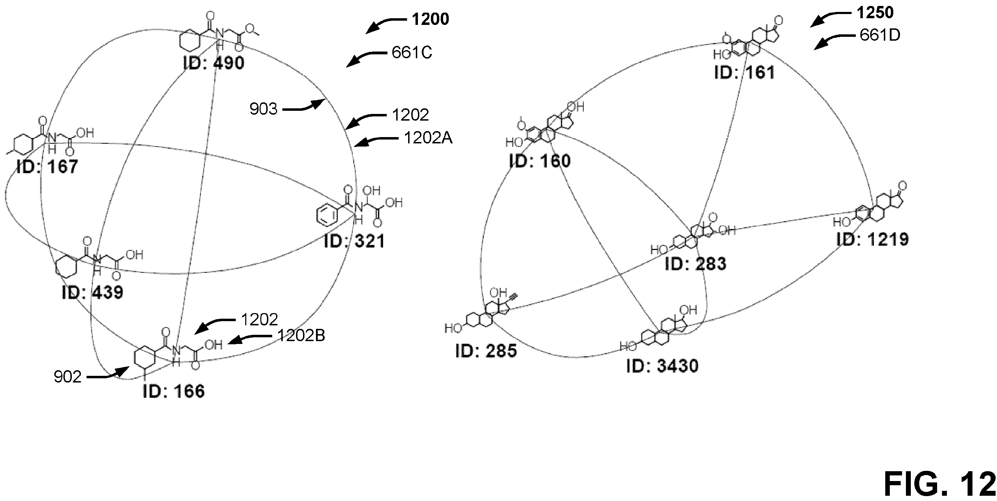

Turning briefly to FIG. 12, the visualizing component 616 can generate a correspondence, tag, label, identifier, metadata, etc., and/or can employ the identification metadata 802, where the relationship can correspond to a respective structural similarity score 650. As illustrated at FIG. 12, an identifier can be employed for one or more nodes 902 or edges 903. For example, a text identifier is employed for nodes 902 at the MN cloud visuals 661C and 661D of illustrations 1200 and 1250 of FIG. 12.

Further, such identifiers can be provided other than by text. For example, nodes 902 and/or edges 903 can be supplemented, by the visualizing component 616 (for generating the underlying data) and/or displaying component 620 (for visualizing the underlying data) with metadata, including compound classes, names, taxonomies, chemical families biochemical activity, and/or hydrophobicity, without being limited thereto, which can be reflected in a visual aspect 1202, such as a size, shape, color, fill color, fill pattern, border color, border thickness, length and/or positioning of a node 902 and/or edge 903. For example, at FIG. 12, a first visual aspect 1202A can comprise a colored edge 903, and a second visual aspect 1202B can comprise a colored molecular structure of a node 902. The coloring can represent any suitable one or more classifications or other representations as described above and/or below, such as at FIG. 13, to be described below.

To provide the visual aspects 1202, the displaying component 620 can evaluate identification metadata 802 of the display data 660, associated with one or more structural molecules 634 and can generate the corresponding visual aspect 1202, based on the evaluation of the identification metadata 802.

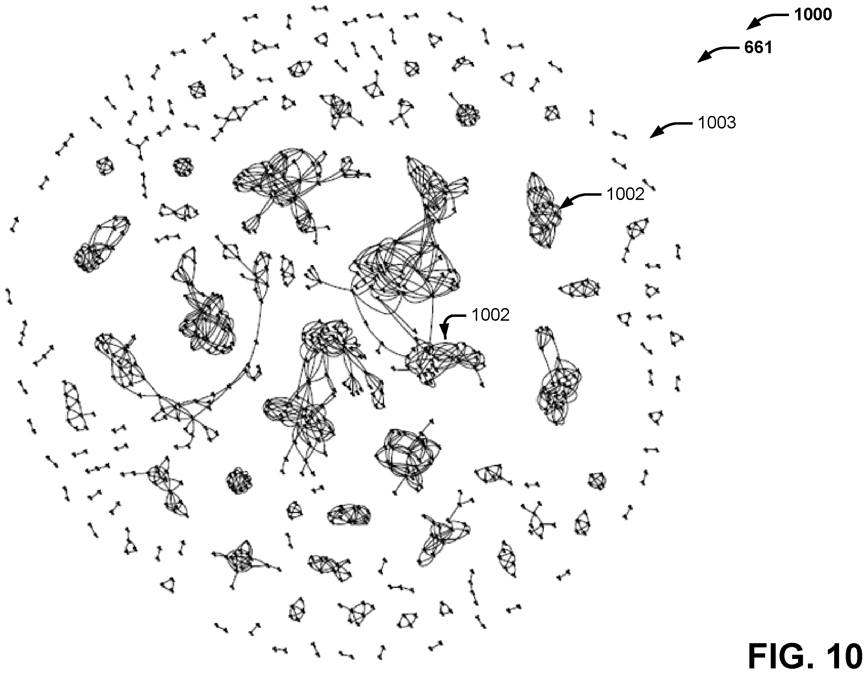

As illustrated at the illustration 1000 of the MN cloud visual 661 of FIG. 10, a plurality of individual clouds 1002 can together be comprised by a parent cloud 1003. In one or more example embodiments, this MN cloud visual 661 can be a two-dimensional visual. In one or more example embodiments, any one or more aspects of a similarity visual 662 can be manipulated, such as by a manipulation 692, such as by a user entity using any suitable instrument, appendage, tactile application, sound application, light application, etc. that is communicable and/or interpretable by a computer device hosting the similarity visual 662. A manipulation 692 can comprise a movement, a re-sizing, or a change in visual aspect (e.g., via a visual modification 690), without being limited thereto.

In one or more example embodiments, the individual clouds 1202 can be moveable relative to one another and/or size adjustable (e.g., zoom in, zoom out, or changing of size without zoom or zoom out) relative to one another. In one or more example embodiments, the individual nodes 902 and/or individual edges 903 can be moveable relative to one another and/or size adjustable (e.g., zoom in, zoom out, or changing of size without zoom or zoom out) relative to one another.

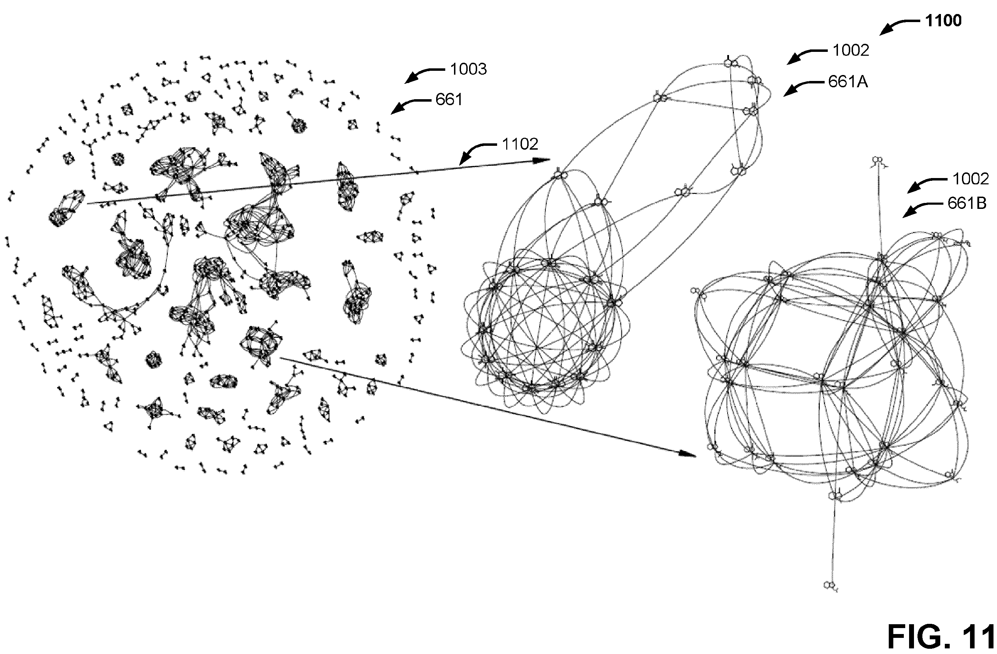

Turning briefly to the MN cloud visual 661 of FIG. 11, which includes the parent cloud 1003 of FIG. 10, in one or more example embodiments, the nodes 902 and/or edges 903 can be clickable, interactable with, interactive, etc., causing change in the molecular structures 634 visualized. For example, selecting a node 902, edge 903, node pair, or individual cloud 1002 can cause that node 902, edge 903, node pair, or individual cloud 1002 to become a center of a cloud visual 661 and/or to bring up a text box including corresponding information (e.g., number of nodes, chemical property, classification, relationship, etc.). For another example, selecting a node 902, edge 903, node pair, or individual cloud 1002 can bring up a text box including definition of a property (e.g., color, thickness, fill, patterning etc.) of the node 902, edge 903, node pair, or individual cloud 1002. For example, selecting an edge 903 can bring up a text box including reasoning or underlying calculation defining the structural similarity score 650 and/or other property (e.g., color, thickness, etc.) of the edge 903.

In one or more example embodiments, one or more tracking arrows or pointers 1102 can be employed between a parent cloud 1003 and an individual cloud 1002 that has been selected and individually illustrated, such as in a size greater than the parent cloud 1003. See, e.g., the pointers 1102 between the individual clouds 661A and 661B selected from the parent cloud 1003 at illustration 1100 of FIG. 11.

In one or more example embodiments, one or more tracking arrows or pointers 1102 can be employed between an individual cloud 1002 and an individual pair of nodes 902 with the respective edge 903 therebetween. In one or more example embodiments, one or more tracking arrows or pointers 1102 can be employed between a parent cloud 1003 and an individual pair of nodes 902 with the respective edge 903 therebetween. In one or more example embodiments, any one or more parent clouds 1003, individual clouds 1002, and/or individual pairs of nodes 902 can be displayed at a same similarity visual 662.

Also, as illustrated at FIG. 11, filtering employed by the filtering component 624, as discussed below, can provide for visual separation of node pairs or individual clouds 1002 into groups, such as spaced apart from one another, using different patterning, border color, etc. Such groups can be of any suitable number and can be based on any suitable parameter or parameters, as will be discussed below relative to FIG. 13.

Turning now to FIG. 13, illustrated is a schematic diagram of an interactive panel GUI 1300 that can be employed to edit and/or filter one or more parameters 1302, 1304, 1306, 1308 and/or 1310, without being limited thereto, employed by the one or more example embodiments described herein to generate a molecular network visualization. The GUI 1300 can be the same as the GUI 300 and/or any description provided above relative to the GUI 300 can be applicable to the GUI 1300.

Parameters that can be optimized can comprise, but are not limited to chemical taxonomies for compounds, color code on nodes, similarity score cutoff, compound representation such as name, formula (ticking dots), number of nodes and generations with type-in windows, and/or number of sharing ions with type-in windows.

For example, general parameters 1302 can comprise number of connections to a node 902, number of nodes to visualize/display, range of similarity scores/edges to employ, etc.

Similarity score basis parameters 1304 can comprise selection of a basis on which the similarity scores 650 are based, e.g., Cosine, HighChem, NIST, Tanimoto, etc.

Multi-class or hierarchical classification parameters 1306 can comprise any suitable ranking or leveling of hierarchies, and/or any suitable set of multi-class classifications suitable for any number of ontologies, whether chemical, classical, biological, functional and/or toxicological. Two or more different such hierarchical classification parameter categories can be employed in one or more example embodiments. For example, a set of multi-class chemical classifications can comprise, but is not limited to, drugs of abuse, natural compounds, surfactants, textile chemicals, extractables, leachables, marine toxins, person care products, cosmetic products, drugs, pesticides, etc.

Visualization parameters 1308 can comprise edge thickness, edge color, edge length, node color, node patterning, node border thickness, node border color and/or node size.

Node ion visualization parameters 1310 can comprise which different types of ions and/or numbers thereof, are to be particularly illustrated rather than generally represented as a letter, number or other general symbol. That is, these parameters can be employed to determine complexity of visualization of fingerprints 630 that are displayed as the nodes 902.

It is noted that use of any of these categories is non-limiting, and indeed, the categories themselves are non-limiting. Any combination of the categories and/or parameters illustrated and/or additional non-illustrated categories and/or parameters can be employed by the visualizing component 616, parameterizing component 622, filtering component 624 and/or displaying component 620, and/or visualized at the interactive property customization GUI 1300 by the displaying component 620.

In one or more example embodiments, these parameters, as illustrated at the interactive property customization GUI 1300, can be modified and/or adjusted by a user entity in combination with the filtering component 624 and/or applied by the parameterizing component 622, in combination with the visualizing component 616 and/or displaying component 620.

For example, the filtering component 624 can redistribute a portion of the set of nodes 902 from a MN cloud visual 661, or even cause addition of new nodes 902 to the MN cloud visual 661, based on selection, at a graphical user interface (e.g., GUI 300 or 1300) displaying the similarity visual 662, of a classification filtering option corresponding to the first molecular structure. These filtering options can be provided in any suitable format and can comprise any one or more of the aforementioned parameters 1302 to 1310, without being limited thereto. As noted, any combination of the categories and/or parameters illustrated and/or additional non-illustrated categories and/or parameters can be employed at least partially at a same time as one another.

Based on the filtering options selected, the filtering component 624 can evaluate the underlying molecular structure data 638 being employed to generated display data 660 of various visualization aspects 1202. Further, based on the filtered molecular structure data 638 filtered by the filtering component 624, the scoring component 614 can generate a set of similarity scores 650 between the nodes 902 to be displayed as a result of the filtering. In one or more cases, such similarity scores 650 can be newly-generated. In one or more cases, only a particular range of similarity scores 650 can be employed, thus by default limiting/filtering the molecular structures 634 being visualized. Any combination of the categories and/or parameters illustrated and/or additional non-illustrated categories and/or parameters can be employed can be stored at the library datastore 635 and accessed by the scoring component 614.

In another example, the parameterizing component 622 can apply a first property of a structural similarity score 650 as a first visual modification 690A of the edge 903 and can apply a second property of the first molecular structure 634A as a second visual modification 690B of the respective node 902 of the first molecular structure 634A.

For another example, the parameterizing component 622 can adjust at least one of the first visual modification 690A or the second visual modification 690B based on selection, at a graphical user interface (e.g., 300 and/or 1300) comprising the similarity visual 662, from a class of properties comprising properties other than at least one of the first property or the second property corresponding to the at least one of the first visual modification 690A or the second visual modification 690B.

In summary, the one or more example embodiments described herein can provide for comparison of molecular structure data 638 with a MN of highly curated molecular structural trees with different metadata taxonomies in once space (e.g., representing the molecular structure data 638), simultaneous visualization of several nearest network families (e.g., plural MN cloud visuals 661) exhibiting molecular structural relationships, and/or customizable visualization options (e.g., as illustrated at FIG. 13).

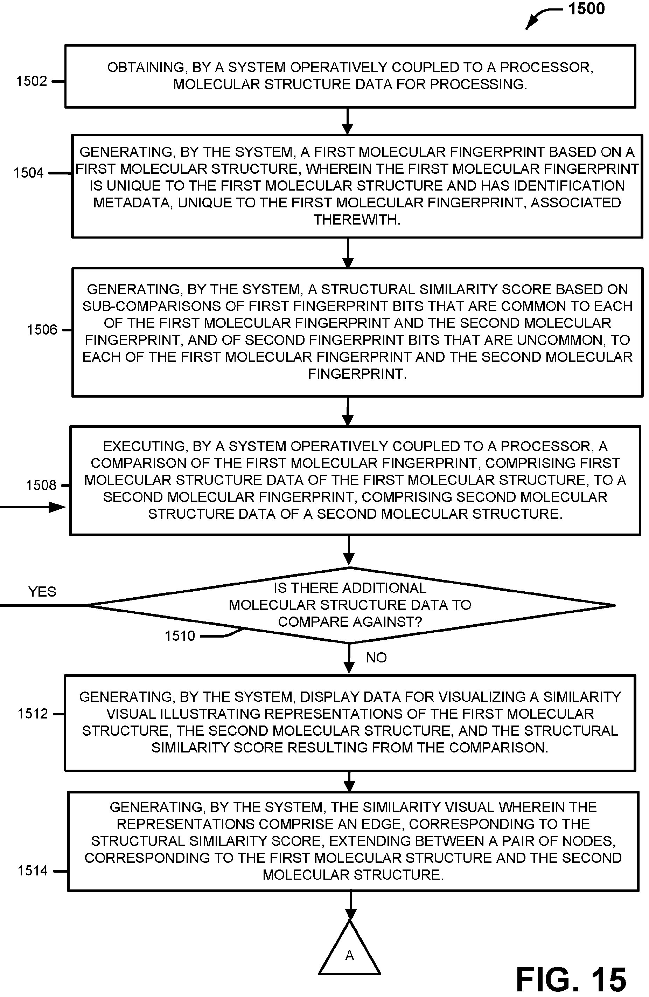

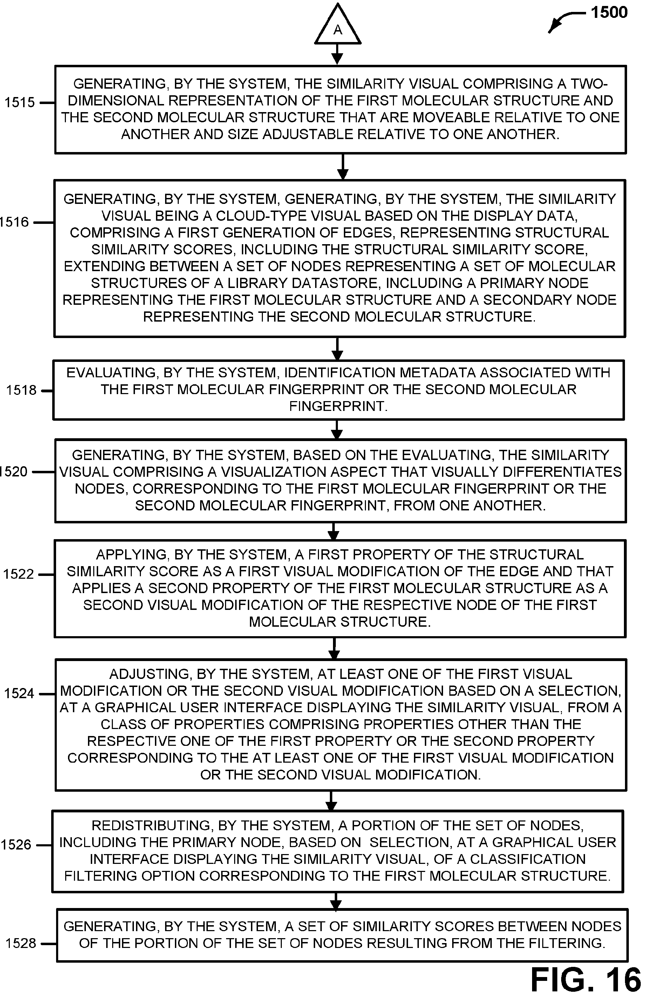

As a summary of the above-described components and/or functions thereof, referring next to FIGS. 15 and 16, illustrated is a flow diagram of an example, non-limiting method 1500 that can facilitate a process for molecular network generation, visualization and/or employment, in accordance with one or more example embodiments described herein, such as the non-limiting system 600 of FIG. 6. While the non-limiting method 1500 is described relative to the non-limiting system 600 of FIG. 6, the non-limiting method 1500 can be applicable also to other systems described herein, such as the non-limiting system 500 of FIG. 5. Repetitive description of like elements and/or processes employed in respective embodiments is omitted for sake of brevity.

At 1502, the non-limiting method 1500 can comprise obtaining, by a system (e.g., obtaining component 610) coupled to a processor (e.g., processor 606), molecular structure data (e.g., molecular structure data 638) for processing.

At 1504, the non-limiting method 1500 can comprise generating, by the system (e.g., fingerprinting component 618), a first molecular fingerprint (e.g., first molecular fingerprint 630A) based on a first molecular structure (e.g., first molecular structure 634A), wherein the first molecular fingerprint is unique to the first molecular structure and has identification metadata (e.g., identification metadata 802), unique to the first molecular fingerprint, associated therewith.

At 1506, the non-limiting method 1500 can comprise generating, by the system (e.g., scoring component 614), a structural similarity score (e.g., structural similarity score 650) based on sub-comparisons of first fingerprint bits (e.g., fingerprint bits 702) that are common to each of the first molecular fingerprint and the second molecular fingerprint, and of second fingerprint bits (e.g., fingerprint bits 702) that are uncommon, to each of the first molecular fingerprint and the second molecular fingerprint.

At 1508, the non-limiting method 1500 can comprise executing, by a system (e.g., evaluating component 612), a comparison of the first molecular fingerprint (e.g., first molecular fingerprint 630A), comprising first molecular structure data (e.g., first molecular structure data 638A) of the first molecular structure (e.g., first molecular structure 634A), to a second molecular fingerprint (e.g., second molecular fingerprint 630B), comprising second molecular structure data (e.g., second molecular structure data 638B) of a second molecular structure (e.g., second molecular structure 634B).

At 1510, determining, by the system (e.g., evaluating component 612), whether there is another known molecular structure data (e.g., known molecular structure data 638) against which to compare the first molecular structure data or the second molecular structure data. If yes, the non-limiting method 1500 can proceed back to step 1508. If not, the non-limiting method can proceed forward to step 1512.

At 1512, the non-limiting method 1500 can comprise generating, by the system (e.g., visualizing component 616) display data (e.g., display data 660) for visualizing a similarity visual (e.g., similarity visual 662) illustrating representations (e.g., representations 664) of the first molecular structure, the second molecular structure, and the structural similarity score resulting from the comparison.

At 1514, the non-limiting method 1500 can comprise generating, by the system (e.g., visualizing component 616), the similarity visual wherein the representations comprise an edge (e.g., edge 903), corresponding to the structural similarity score, extending between a pair of nodes (e.g., nodes 902), corresponding to the first molecular structure and the second molecular structure.

At 1515, the non-limiting method 1500 can comprise generating, by the system (e.g., displaying component 620), the similarity visual comprising a two-dimensional representation of the first molecular structure and the second molecular structure that are moveable relative to one another and size adjustable relative to one another.

At 1516, the non-limiting method 1500 can comprise generating, by the system (e.g., displaying component 620), the similarity visual being a cloud-type visual (e.g., MN cloud visual 661) based on the display data (e.g., display data 660), comprising a first generation of edges, representing structural similarity scores, including the structural similarity score, extending between a set of nodes representing a set of molecular structures of a library datastore (e.g., library datastore 635), including a primary node representing the first molecular structure and a secondary node representing the second molecular structure.

At 1518, the non-limiting method 1500 can comprise evaluating, by the system (e.g., displaying component 620), identification metadata (e.g., identification metadata 802) associated with the first molecular fingerprint or the second molecular fingerprint.

At 1520, the non-limiting method 1500 can comprise generating, by the system (e.g., displaying component 620), based on the evaluating, the similarity visual comprising a visualization aspect (e.g., visualization aspect 1202) that visually differentiates nodes, corresponding to the first molecular fingerprint or the second molecular fingerprint, from one another.

At 1522, the non-limiting method 1500 can comprise applying, by the system (e.g., parameterizing component 622), a first property of the structural similarity score as a first visual modification of the edge and that applies a second property of the first molecular structure as a second visual modification of the respective node of the first molecular structure.

At 1524, the non-limiting method 1500 can comprise adjusting, by the system (e.g., parameterizing component 622), at least one of the first visual modification or the second visual modification based on a selection, at a graphical user interface (e.g., GUI 300 or 1300) displaying the similarity visual, from a class of properties comprising properties other than the respective one of the first property or the second property corresponding to the at least one of the first visual modification or the second visual modification (see, e.g., various properties at FIG. 13).

At 1526, the non-limiting method 1500 can comprise redistributing, by the system (e.g., filtering component 624), a portion of the set of nodes, including the primary node, based on selection, at a graphical user interface displaying the similarity visual, of a classification filtering option corresponding to the first molecular structure.

At 1528, the non-limiting method 1500 can comprise generating, by the system (e.g., scoring component 614), a set of similarity scores between nodes of the portion of the set of nodes resulting from the filtering.

Additional Summary

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. In addition, the computer-implemented and non-computer-implemented methodologies could alternatively be represented as a series of interrelated states via a state diagram or events. Additionally, the computer-implemented methodologies described hereinafter and throughout this specification are capable of being stored on an article of manufacture for transporting and transferring the computer-implemented methodologies to computers. The term article of manufacture, as used herein, is intended to encompass a computer program accessible from any computer-readable device or storage media.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

In summary, one or more systems, computer program products and/or computer-implemented methods provided herein described herein relate to a process for molecular network generation based on molecular structural content. A system can comprise a memory (e.g., memory 504, 604) that stores, and a processor (e.g., processor 506, 606) that executes, computer executable components. The computer executable components can comprise an evaluating component (e.g., evaluating component 512, 612) that executes a comparison of a first molecular fingerprint (e.g., first molecular fingerprint 530A, 630A), comprising first molecular structure data (e.g., first molecular structure data 538A, 638A) of a first molecular structure (e.g., first molecular structure 534A, 634A), to a second molecular fingerprint (e.g., second molecular fingerprint 530B 630B), comprising second molecular structure data (e.g., second molecular structure data 538B, 638B) of a second molecular structure (e.g., second molecular structure 534B, 634B), and a visualizing component (e.g., visualizing component 516, 616) that generates display data (e.g., display data 560, 660) for visualizing a similarity visual (e.g., similarity visual 562, 662) illustrating representations (e.g., representations 564, 664) of the first molecular structure (e.g., first molecular structure 534A, 634A), the second molecular structure (e.g., second molecular structure 534B, 634B), and a structural similarity score (e.g., structural similarity score 550, 650) resulting from the comparison. The representations can comprise an edge (e.g., edge 903), corresponding to the structural similarity score (e.g., structural similarity score 550, 650), extending between a pair of nodes (e.g., nodes 902), corresponding to the first molecular structure (e.g., first molecular structure 534A, 634A) and the second molecular structure (e.g., second molecular structure 534B, 634B).

The one or more example embodiments described herein can be employed to generate a molecular network that can provide varying visual, configurable and/or interactive outputs during use of the molecular network. For example, based on visual aspects of a format of a MN cloud, such as coloring, line thicknesses, shapes and/or distances between different aspects of the MN cloud, the system can illustrate one or more chemical properties and/or relationships corresponding to the molecular structural content underlying the MN cloud. These one or more chemical properties and/or relationships can comprise chemical class, chemical use, similar compounds, etc.

The one or more example embodiments described herein can provide the molecular network visual being a dynamically adjustable visual that can provide varied visualization types and/or customization of visualized chemical relationships and/or properties. For example, dynamic adjustability can be found in functioning of the generated molecular network (MN), where a user entity can interact with the visual display to vary chemical classes, chemical properties, sizes and/or distances of varying MN aspects, etc. Varied visualizations can comprise large MN clouds, customized clouds based on one or more specified parameters, plural clouds displayed at a same time as one another, etc. Customization can be provided by use of a graphical user interface (GUI) allowing for different chemical properties and/or relationships to be represented by nodes, edges, borders of nodes and/or edges, fill of nodes and/or edges, thickness of lines within a cloud, distances between nodes, etc.

The one or more example embodiments described herein can be implemented within, in connection with and/or coupled to a scientific imaging device.

The one or more example embodiments disclosed herein can be applied on a plug-and-play basis to various architectures of existing molecular structural library and/or library datastores of molecular structural data. That is, the one or more example embodiments described herein can generate and/or update a molecular network comprising a visual representing a plurality of chemical relationships regardless of data structure of a molecular structural library.

Indeed, in view of the one or more example embodiments described herein, a practical application of the one or more systems, computer-implemented methods and/or computer program products described herein can be ability to provide the aforementioned dynamically adjustable visual representation of relationships between molecular structural content of the molecular structural library. That is, a molecular network visual can be realized and displayed, which visual can allow for an understanding of a chemical property, relationship and/or classification of and/or corresponding to the molecular structures being selectively displayed (e.g., based on filtering options). As compared to existing frameworks that cannot provide this ability for molecular structural content, the one or more example embodiments described herein can provide a new result that was previously unavailable.

These are useful and practical applications of computers, thus providing enhanced (e.g., improved and/or optimized) molecular analysis and/or molecular structural analysis output. Overall, such computerized tools can constitute a concrete and tangible technical improvement in the fields of material analysis, and more particularly in material analysis using molecular networks and/or molecular network cloud visuals generated therefrom.

Furthermore, one or more example embodiments described herein can be employed in a real-world system based on the disclosed teachings. For example, a molecular structural library datastore of molecular structural content can be identified and the content evaluated. Based thereon, a molecular network can be visually generated comprising one or more molecular network cloud visuals (and data underlying the cloud visuals) which illustrate one or more chemical correspondences (e.g., chemical properties, relationships and/or classification) for the one or more molecular structures being represented. These can be useful processes for varying industries employing material analysis, product manufacturing, quality control and/or the like. The embodiments disclosed herein thus can provide improvements to scientific instrument technology (e.g., improvements in the computer technology supporting such scientific instruments, among other improvements).

Moreover, the one or more example embodiments described herein can achieve a level of scale of operation. For example, two or more library databases of molecular structural content can be analyzed and one or more corresponding molecular networks can be generated based thereon, at least partially in parallel with one another. In one or more cases, two or more MN cloud visuals can be generated at least partially at a same time as one another.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

One or more example embodiments described herein can be, in one or more example embodiments, inherently and/or inextricably tied to computer technology and cannot be implemented outside of a computing environment. For example, one or more processes performed by one or more example embodiments described herein can more efficiently, and even more feasibly, provide program and/or program instruction execution, such as relative to material analysis using molecular network generation and/or visualization, as compared to existing systems and/or techniques using molecular network generation and/or visualization. Systems, computer-implemented methods and/or computer program products providing performance of these processes are of great utility in the fields of material analysis, such for visually illustrating one or more chemical correspondences (e.g., chemical properties, relationships and/or classification) for a set of molecular structural content and cannot be equally practicably implemented in a sensible way outside of a computing environment.

One or more example embodiments described herein can employ hardware and/or software to solve problems that are highly technical, that are not abstract, and that cannot be performed as a set of mental acts by a human. For example, a human, or even thousands of humans, cannot efficiently, accurately and/or effectively analyze computer data/metadata defining molecular structural content for a plurality of compounds, and/or generate a digital display visual of a molecular network based on a plurality of known molecular structural data, while employing a plurality of different chemical correspondences to bound and/or adjust the display visual as the one or more example embodiments described herein can provide this process. Moreover, neither can the human mind nor a human with pen and paper conduct one or more of these processes, as conducted by one or more example embodiments described herein.

In one or more example embodiments, one or more of the processes described herein can be performed by one or more specialized computers (e.g., a specialized processing unit, a specialized classical computer, a specialized quantum computer, a specialized hybrid classical/quantum system and/or another type of specialized computer) to execute defined tasks related to the one or more technologies describe above. One or more example embodiments described herein and/or components thereof can be employed to solve new problems that arise through advancements in technologies mentioned above, employment of quantum computing systems, cloud computing systems, computer architecture and/or another technology.

One or more example embodiments described herein can be fully operational towards performing one or more other functions (e.g., fully powered on, fully executed and/or another function) while also performing one or more of the one or more operations described herein.

To provide additional summary, a listing of embodiments and features thereof is next provided.

A system, comprising: a memory that stores computer executable components; and a processor that executes the computer executable components stored in the memory, wherein the computer executable components comprise: an evaluating component that executes a comparison of a first molecular fingerprint, comprising first molecular structure data of a first molecular structure, to a second molecular fingerprint, comprising second molecular structure data of a second molecular structure; and a visualizing component that generates display data for visualizing a similarity visual illustrating representations of the first molecular structure, the second molecular structure, and a structural similarity score resulting from the comparison.

The system of the preceding paragraph, wherein the representations comprise an edge, corresponding to the structural similarity score, extending between a pair of nodes, corresponding to the first molecular structure and the second molecular structure.

The system of any preceding paragraph, wherein the computer executable components further comprise: a parameterizing component that applies a first property of the structural similarity score as a first visual modification of the edge and that applies a second property of the first molecular structure as a second visual modification of the respective node of the first

The system of any preceding paragraph, wherein the parameterizing component adjusts at least one of the first visual modification or the second visual modification based on a selection, at a graphical user interface displaying the similarity visual, from a class of properties comprising properties other than the respective one of the first property or the second property corresponding to the at least one of the first visual modification or the second visual modification.

The system of any preceding paragraph, wherein the computer executable components further comprise: a scoring component that generates the structural similarity score based on sub-comparisons of first fingerprint bits that are common to each of the first molecular fingerprint and the second molecular fingerprint, and of second fingerprint bits that are uncommon, to each of the first molecular fingerprint and the second molecular fingerprint.

The system of any preceding paragraph, wherein the computer executable components further comprise: a fingerprinting component that generates the first molecular fingerprint based on the first molecular structure, wherein the first molecular fingerprint is unique to the first molecular structure and has identification metadata, unique to the first molecular fingerprint, associated therewith.

The system of any preceding paragraph, wherein the computer executable components further comprise: a displaying component that evaluates identification metadata associated with the first molecular fingerprint or the second molecular fingerprint and generates, based on the evaluating, the similarity visual comprising a visualization aspect that visually differentiates nodes, corresponding to the first molecular fingerprint or the second molecular fingerprint, from one another.

The system of any preceding paragraph, wherein the computer executable components further comprise: a displaying component that generates the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

The system of any preceding paragraph, wherein the computer executable components further comprise: a filtering component that redistributes a portion of the set of nodes, including the primary node, based on selection, at a graphical user interface displaying the similarity visual, of a classification filtering option corresponding to the first molecular structure, wherein the scoring component generates a set of similarity scores between nodes of the portion of the set of nodes resulting from the filtering.

The system of any preceding paragraph, wherein the similarity visual comprises a two-dimensional representation of the first molecular structure and the second molecular structure that are moveable relative to one another and size adjustable relative to one another.

A computer-implemented method, comprising: executing, by a system operatively coupled to a processor, a comparison of a first molecular fingerprint, comprising first molecular structure data of a first molecular structure, to a second molecular fingerprint, comprising second molecular structure data of a second molecular structure; and generating, by the system, display data for visualizing a similarity visual illustrating representations of the first molecular structure, the second molecular structure, and a structural similarity score resulting from the comparison.

The computer-implemented method of the preceding paragraph, wherein the representations comprise an edge, corresponding to the structural similarity score, extending between a pair of nodes, corresponding to the first molecular structure and the second molecular structure.

The computer-implemented method of any preceding paragraph, further comprising: applying, by the system, a first property of the structural similarity score as a first visual modification of the edge and that applies a second property of the first molecular structure as a second visual modification of the respective node of the first molecular structure.

The computer-implemented method of any preceding paragraph, further comprising: evaluating, by the system, identification metadata associated with the first molecular fingerprint or the second molecular fingerprint; and generating, by the system, based on the evaluating, the similarity visual comprising a visualization aspect that visually differentiates nodes, corresponding to the first molecular fingerprint or the second molecular fingerprint, from one another.

The computer-implemented method of any preceding paragraph, further comprising: generating, by the system, the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

The computer-implemented method of any preceding paragraph, further comprising: redistributing, by the system, a portion of the set of nodes, including the primary node, based on selection, at a graphical user interface displaying the similarity visual, of a classification filtering option corresponding to the first molecular structure; and generating, by the system, a set of similarity scores between nodes of the portion of the set of nodes resulting from the filtering.

A computer program product facilitating a process for visualizing and comparing molecular structures, the computer program product comprising a computer readable storage medium having program instructions embodied therewith, and the program instructions executable by a processor to cause the processor to: execute, by the processor, a comparison of a first molecular fingerprint, comprising first molecular structure data of a first molecular structure, to a second molecular fingerprint, comprising second molecular structure data of a second molecular structure; and generate, by the processor, display data for visualizing a similarity visual illustrating representations of the first molecular structure, the second molecular structure, and a structural similarity score resulting from the comparison.

The computer program product of the preceding paragraph, wherein the representations comprise an edge, corresponding to the structural similarity score, extending between a pair of nodes, corresponding to the first molecular structure and the second molecular structure.

The computer program product of any preceding paragraph, wherein the program instructions are further executable by the processor to cause the processor to: evaluate, by the processor, identification metadata associated with the first molecular fingerprint or the second molecular fingerprint; and generate, by the processor, based on the evaluating, the similarity visual comprising a visualization aspect that visually differentiates nodes, corresponding to the first molecular fingerprint or the second molecular fingerprint, from one another.

The computer program product of any preceding paragraph, wherein the program instructions are further executable by the processor to cause the processor to: generate, by the processor, the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

The computer program product of any preceding paragraph, wherein the program instructions are further executable by the processor to cause the processor to: redistribute, by the processor, a portion of the set of nodes, including the primary node, based on selection, at a graphical user interface displaying the similarity visual, of a classification filtering option corresponding to the first molecular structure; and generate, by the processor, a set of similarity scores between nodes of the portion of the set of nodes resulting from the filtering.

Scientific Instrument System Description

Turning next to FIG. 17, a detailed description is provided of additional context for the one or more example embodiments described herein at FIGS. 1-16. One or more computing devices implementing any of the scientific instrument modules or methods disclosed herein can be part of a scientific instrument system. FIG. 17 illustrates a block diagram of an example scientific instrument system 1700 in which one or more of the scientific instrument methods or other methods disclosed herein can be performed, in accordance with various embodiments described herein. The scientific instrument modules and methods disclosed herein (e.g., the scientific instrument module 100 of FIG. 1 and the method 200 of FIG. 2) can be implemented by one or more of the scientific instrument 1710, the user local computing device 1720, the service local computing device 1730, and/or the remote computing device 1740 of the scientific instrument system 1700.

Any of the scientific instrument 1710, the user local computing device 1720, the service local computing device 1730, and/or the remote computing device 1740 can include any of the embodiments of the computing device 400 discussed herein with reference to FIG. 4, and any of the scientific instrument 1710, the user local computing device 1720, the service local computing device 1730, and/or the remote computing device 1740 can take the form of any appropriate one or more of the embodiments of the computing device 400 discussed herein with reference to FIG. 4.

One or more of the scientific instrument 1710, the user local computing device 1720, the service local computing device 1730, and/or the remote computing device 1740 can include a processing device 1702, a storage device 1704, and/or an interface device 1706. The processing device 1702 can take any suitable form, including the form of any of the processors 402 discussed herein with reference to FIG. 4. The processing devices 1702 included in different ones of the scientific instrument 1710, the user local computing device 1720, the service local computing device 1730, and/or the remote computing device 1740 can take the same form or different forms. The storage device 1704 can take any suitable form, including the form of any of the storage devices 404 discussed herein with reference to FIG. 4. The storage devices 1704 included in different ones of the scientific instrument 1710, the user local computing device 1720, the service local computing device 1730, and/or the remote computing device 1740 can take the same form or different forms. The interface device 1706 can take any suitable form, including the form of any of the interface devices 406 discussed herein with reference to FIG. 4. The interface devices 1706 included in different ones of the scientific instrument 1710, the user local computing device 1720, the service local computing device 1730, and/or the remote computing device 1740 can take the same form or different forms.

The scientific instrument 1710, the user local computing device 1720, the service local computing device 1730, and/or the remote computing device 1740 can be in communication with other elements of the scientific instrument system 1700 via communication pathways 1708. The communication pathways 1708 can communicatively couple the interface devices 1706 of different ones of the elements of the scientific instrument system 1700, as shown, and can be wired or wireless communication pathways (e.g., in accordance with any of the communication techniques discussed herein with reference to the interface devices 406 of the computing device 400 of FIG. 4). The particular scientific instrument system 1700 depicted in FIG. 17 includes communication pathways between each pair of the scientific instrument 1710, the user local computing device 1720, the service local computing device 1730, and the remote computing device 1740, but this "fully connected" implementation is simply illustrative, and in various embodiments, various ones of the communication pathways 1708 can be omitted. For example, in one or more example embodiments, a service local computing device 1730 can omit a direct communication pathway 1708 between its interface device 1706 and the interface device 1706 of the scientific instrument 1710, but can instead communicate with the scientific instrument 1710 via the communication pathway 1708 between the service local computing device 1730 and the user local computing device 1720 and/or the communication pathway 1708 between the user local computing device 1720 and the scientific instrument 1710.

The scientific instrument 1710 can include any appropriate scientific instrument, such as a separation or MS instrument, or other instrument facilitating material analysis.

The user local computing device 1720 can be a computing device (e.g., in accordance with any of the embodiments of the computing device 400 discussed herein) that is local to a user of the scientific instrument 1710. In one or more example embodiments, the user local computing device 1720 can also be local to the scientific instrument 1710, but this need not be the case; for example, a user local computing device 1720 that is associated with a home, office or other building associated with a user entity can be remote from, but in communication with, the scientific instrument 1710 so that the user entity can use the user local computing device 1720 to control and/or access data from the scientific instrument 1710. In one or more example embodiments, the user local computing device 1720 can be a laptop, smartphone, or tablet device. In one or more example embodiments the user local computing device 1720 can be a portable computing device. In one or more example embodiments, the user local computing device 1720 can deployed in the field.

The service local computing device 1730 can be a computing device (e.g., in accordance with any of the embodiments of the computing device 400 discussed herein) that is local to an entity that services the scientific instrument 1710. For example, the service local computing device 1730 can be local to a manufacturer of the scientific instrument 1710 or to a third-party service company. In one or more example embodiments, the service local computing device 1730 can communicate with the scientific instrument 1710, the user local computing device 1720, and/or the remote computing device 1740 (e.g., via a direct communication pathway 1708 or via multiple "indirect" communication pathways 1708, as discussed above) to receive data regarding the operation of the scientific instrument 1710, the user local computing device 1720, and/or the remote computing device 1740 (e.g., the results of self-tests of the scientific instrument 1710, calibration coefficients used by the scientific instrument 1710, the measurements of sensors associated with the scientific instrument 1710, etc.). In one or more example embodiments, the service local computing device 1730 can communicate with the scientific instrument 1710, the user local computing device 1720, and/or the remote computing device 1740 (e.g., via a direct communication pathway 1708 or via multiple "indirect" communication pathways 1708, as discussed above) to transmit data to the scientific instrument 1710, the user local computing device 1720, and/or the remote computing device 1740 (e.g., to update programmed instructions, such as firmware, in the scientific instrument 1710, to initiate the performance of test or calibration sequences in the scientific instrument 1710, to update programmed instructions, such as software, in the user local computing device 1720 or the remote computing device 1740, etc.). A user entity of the scientific instrument 1710 can utilize the scientific instrument 1710 or the user local computing device 1720 to communicate with the service local computing device 1730 to report a problem with the scientific instrument 1710 or the user local computing device 1720, to request a visit from a technician to improve the operation of the scientific instrument 1710, to order consumables or replacement parts associated with the scientific instrument 1710, or for other purposes.

The remote computing device 1740 can be a computing device (e.g., in accordance with any of the embodiments of the computing device 400 discussed herein) that is remote from the scientific instrument 1710 and/or from the user local computing device 1720. In one or more example embodiments, the remote computing device 1740 can be included in a datacenter or other large-scale server environment. In one or more example embodiments, the remote computing device 1740 can include network-attached storage (e.g., as part of the storage device 1704). The remote computing device 1740 can store data generated by the scientific instrument 1710, perform analyses of the data generated by the scientific instrument 1710 (e.g., in accordance with programmed instructions), facilitate communication between the user local computing device 1720 and the scientific instrument 1710, and/or facilitate communication between the service local computing device 1730 and the scientific instrument 1710.

In one or more example embodiments, one or more of the elements of the scientific instrument system 1700 illustrated in FIG. 17 can be omitted. Further, in one or more example embodiments, multiple ones of various ones of the elements of the scientific instrument system 1700 of FIG. 17 can be present. For example, a scientific instrument system 1700 can include multiple user local computing devices 1720 (e.g., different user local computing devices 1720 associated with different user entities or in different locations). In another example, a scientific instrument system 1700 can include multiple scientific instruments 1710, all in communication with service local computing device 1730 and/or a remote computing device 1740; in such an embodiment, the service local computing device 1730 can monitor these multiple scientific instruments 1710, and the service local computing device 1730 can cause updates or other information can be "broadcast" to multiple scientific instruments 1710 at the same time. Different ones of the scientific instruments 1710 in a scientific instrument system 1700 can be located close to one another (e.g., in the same room) or farther from one another (e.g., on different floors of a building, in different buildings, in different cities, etc.). In one or more example embodiments, a scientific instrument 1710 can be connected to an Internet-of-Things (IoT) stack that allows for command and control of the scientific instrument 1710 through a web-based application, a virtual or augmented reality application, a mobile application, and/or a desktop application. Any of these applications can be accessed by a user entity operating the user local computing device 1720 in communication with the scientific instrument 1710 by the intervening remote computing device 1740. In one or more example embodiments, a scientific instrument 1710 can be sold by the manufacturer along with one or more associated user local computing devices 1720 as part of a local scientific instrument computing unit 1712.

In one or more example embodiments, different ones of the scientific instruments 1710 included in a scientific instrument system 1700 can be different types of scientific instruments 1710; for example, one scientific instrument 1710 can be an EDS device, while another scientific instrument 1710 can be an analysis device that analyzes results of an EDS device. In some such embodiments, the remote computing device 1740 and/or the user local computing device 1720 can combine data from different types of scientific instruments 1710 included in a scientific instrument system 1700.

Example Operating Environment

FIG. 18 is a schematic block diagram of an operating environment 1800 with which the described subject matter can interact. The operating environment 1800 comprises one or more remote component(s) 1810. The remote component(s) 1810 can be hardware and/or software (e.g., threads, processes, computing devices). In one or more example embodiments, remote component(s) 1810 can be a distributed computer system, connected to a local automatic scaling component and/or programs that use the resources of a distributed computer system, via communication framework 1840. Communication framework 1840 can comprise wired network devices, wireless network devices, mobile devices, wearable devices, radio access network devices, gateway devices, femtocell devices, servers, etc.

The operating environment 1800 also comprises one or more local component(s) 1820. The local component(s) 1820 can be hardware and/or software (e.g., threads, processes, computing devices). In one or more example embodiments, local component(s) 1820 can comprise an automatic scaling component and/or programs that communicate/use the remote resources 1810 and 1820, etc., connected to a remotely located distributed computing system via communication framework 1840.

One possible communication between a remote component(s) 1810 and a local component(s) 1820 can be in the form of a data packet adapted to be transmitted between two or more computer processes. Another possible communication between a remote component(s) 1810 and a local component(s) 1820 can be in the form of circuit-switched data adapted to be transmitted between two or more computer processes in radio time slots. The operating environment 1800 comprises a communication framework 1840 that can be employed to facilitate communications between the remote component(s) 1810 and the local component(s) 1820, and can comprise an air interface, e.g., interface of a UMTS network, via an LTE network, etc. Remote component(s) 1810 can be operably connected to one or more remote data store(s) 1850, such as a hard drive, solid state drive, subscriber identity module (SIM) card, electronic SIM (eSIM), device memory, etc., that can be employed to store information on the remote component(s) 1810 side of communication framework 1840. Similarly, local component(s) 1820 can be operably connected to one or more local data store(s) 1830, that can be employed to store information on the local component(s) 1820 side of communication framework 1840.

Example Computing Environment

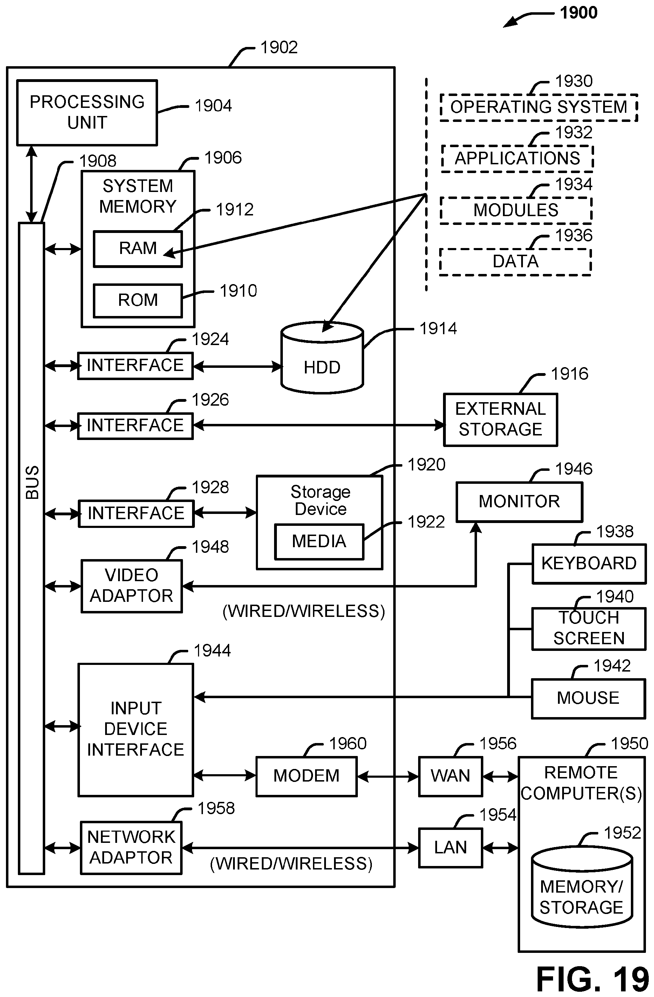

In order to provide additional context for various embodiments described herein, FIG. 19 and the following discussion are intended to provide a brief, general description of a suitable computing environment 1900 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform tasks or implement abstract data types. Moreover, the methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can also be practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

Referring still to FIG. 19, the example computing environment 1900 which can implement one or more example embodiments described herein includes a computer 1902, the computer 1902 including a processing unit 1904, a system memory 1906 and a system bus 1908. The system bus 1908 couples system components including, but not limited to, the system memory 1906 to the processing unit 1904. The processing unit 1904 can be any of various commercially available processors. Dual microprocessors and other multi processor architectures can also be employed as the processing unit 1904.

The system bus 1908 can be any of several types of bus structure that can further interconnect to a memory bus (with or without a memory controller), a peripheral bus, and a local bus using any of a variety of commercially available bus architectures. The system memory 1906 includes ROM 1910 and RAM 1912. A basic input/output system (BIOS) can be stored in a nonvolatile memory such as ROM, erasable programmable read only memory (EPROM), EEPROM, which BIOS contains the basic routines that help to transfer information between elements within the computer 1902, such as during startup. The RAM 1912 can also include a high-speed RAM such as static RAM for caching data.

The computer 1902 further includes an internal hard disk drive (HDD) 1914 (e.g., EIDE, SATA), and can include one or more external storage devices 1916 (e.g., a magnetic floppy disk drive (FDD) 1916, a memory stick or flash drive reader, a memory card reader, etc.). While the internal HDD 1914 is illustrated as located within the computer 1902, the internal HDD 1914 can also be configured for external use in a suitable chassis (not shown). Additionally, while not shown in computing environment 1900, a solid-state drive (SSD) could be used in addition to, or in place of, an HDD 1914.

Other internal or external storage can include at least one other storage device 1920 with storage media 1922 (e.g., a solid-state storage device, a nonvolatile memory device, and/or an optical disk drive that can read or write from removable media such as a CD-ROM disc, a DVD, a BD, etc.). The external storage 1916 can be facilitated by a network virtual machine. The HDD 1914, external storage device 1916 and storage device (e.g., drive) 1920 can be connected to the system bus 1908 by an HDD interface 1924, an external storage interface 1926 and a drive interface 1928, respectively.

The drives and their associated computer-readable storage media provide nonvolatile storage of data, data structures, computer-executable instructions, and so forth. For the computer 1902, the drives and storage media accommodate the storage of any data in a suitable digital format. Although the description of computer-readable storage media above refers to respective types of storage devices, other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1912, including an operating system 1930, one or more application programs 1932, other program modules 1934 and program data 1936. All or portions of the operating system, applications, modules, and/or data can also be cached in the RAM 1912. The systems and methods described herein can be implemented utilizing various commercially available operating systems or combinations of operating systems.

Computer 1902 can optionally comprise emulation technologies. For example, a hypervisor (not shown) or other intermediary can emulate a hardware environment for operating system 1930, and the emulated hardware can optionally be different from the hardware illustrated in FIG. 19. In such an embodiment, operating system 1930 can comprise one virtual machine (VM) of multiple VMs hosted at computer 1902. Furthermore, operating system 1930 can provide runtime environments, such as the Java runtime environment or the .NET framework, for applications 1932. Runtime environments are consistent execution environments that allow applications 1932 to run on any operating system that includes the runtime environment. Similarly, operating system 1930 can support containers, and applications 1932 can be in the form of containers, which are lightweight, standalone, executable packages of software that include, e.g., code, runtime, system tools, system libraries and settings for an application.

Further, computer 1902 can be enabled with a security module, such as a trusted processing module (TPM). For instance, with a TPM, boot components hash next in time boot components, and wait for a match of results to secured values, before loading a next boot component. This process can take place at any layer in the code execution stack of computer 1902, e.g., applied at the application execution level or at the operating system (OS) kernel level, thereby enabling security at any level of code execution.

A user entity can enter commands and information into the computer 1902 through one or more wired/-wireless input devices, e.g., a keyboard 1938, a touch screen 1940, and a pointing device, such as a mouse 1942. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera, a gesture sensor input device, a vision movement sensor input device, an emotion or facial detection device, a biometric input device, e.g., fingerprint or iris scanner, or the like. These and other input devices are often connected to the processing unit 1904 through an input device interface 1944 that can be coupled to the system bus 1908, but can be connected by other interfaces, such as a parallel port, an IEEE 1394 serial port, a game port, a USB port, an IR interface, a BLUETOOTH® interface, etc.

A monitor 1946 or other type of display device can also be connected to the system bus 1908 via an interface, such as a video adapter 1948. In addition to the monitor 1946, a computer typically includes other peripheral output devices (not shown), such as speakers, printers, etc.

The computer 1902 can operate in a networked environment using logical connections via wired and/or wireless communications to one or more remote computers, such as a remote computer 1950. The remote computer 1950 can be a workstation, a server computer, a router, a personal computer, portable computer, microprocessor-based entertainment appliance, a peer device or other common network node, and typically includes many or all of the elements described relative to the computer 1902, although, for purposes of brevity, only a memory/storage device 1952 is illustrated. The logical connections depicted include wired/wireless connectivity to a local area network (LAN) 1954 and/or larger networks, e.g., a wide area network (WAN) 1956. Such LAN and WAN networking environments are commonplace in offices and companies, and facilitate enterprise-wide computer networks, such as intranets, all of which can connect to a global communications network, e.g., the Internet.

When used in a LAN networking environment, the computer 1902 can be connected to the local network 1954 through a wired and/or wireless communication network interface or adapter 1958. The adapter 1958 can facilitate wired or wireless communication to the LAN 1954, which can also include a wireless access point (AP) disposed thereon for communicating with the adapter 1958 in a wireless mode.

When used in a WAN networking environment, the computer 1902 can include a modem 1960 or can be connected to a communications server on the WAN 1956 via other means for establishing communications over the WAN 1956, such as by way of the Internet. The modem 1960, which can be internal or external and a wired or wireless device, can be connected to the system bus 1908 via the input device interface 1944. In a networked environment, program modules depicted relative to the computer 1902 or portions thereof, can be stored in the remote memory/storage device 1952. The network connections shown are example and other means of establishing a communications link between the computers can be used.

When used in either a LAN or WAN networking environment, the computer 1902 can access cloud storage systems or other network-based storage systems in addition to, or in place of, external storage devices 1916 as described above. Generally, a connection between the computer 1902 and a cloud storage system can be established over a LAN 1954 or WAN 1956 e.g., by the adapter 1958 or modem 1960, respectively. Upon connecting the computer 1902 to an associated cloud storage system, the external storage interface 1926 can, with the aid of the adapter 1958 and/or modem 1960, manage storage provided by the cloud storage system as it would other types of external storage. For instance, the external storage interface 1926 can be configured to provide access to cloud storage sources as if those sources were physically connected to the computer 1902.

The computer 1902 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a defined structure as with an existing network or simply an ad hoc communication between at least two devices.

Additional Information

The embodiments described herein can be directed to one or more of a system, a method, an apparatus and/or a computer program product at any possible technical detail level of integration. The computer program product can include a computer readable storage medium (or media) having computer readable program instructions thereon for causing a processor to carry out aspects of the one or more example embodiments described herein. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a superconducting storage device and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, is not to be construed as being transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein can be downloaded to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more example embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more example embodiments, electronic circuitry including, for example, programmable logic circuitry, field-programmable gate arrays (FPGA) and/or programmable logic arrays (PLA) can execute the computer readable program instructions by utilizing state information of the computer readable program instructions to personalize the electronic circuitry, in order to perform aspects of the one or more example embodiments described herein.

Aspects of the one or more example embodiments described herein are described with reference to flowchart illustrations and/or block diagrams of methods, apparatus (systems), and computer program products according to one or more example embodiments described herein. It will be understood that each block of the flowchart illustrations and/or block diagrams, and combinations of blocks in the flowchart illustrations and/or block diagrams, can be implemented by computer readable program instructions. These computer readable program instructions can be provided to a processor of a general-purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more example embodiments described herein. In this regard, each block in the flowchart or block diagrams can represent a module, segment and/or portion of instructions, which comprises one or more executable instructions for implementing the specified logical function. In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter has been described above in the general context of computer-executable instructions of a computer program product that runs on a computer and/or computers, those skilled in the art will recognize that the one or more example embodiments herein also can be implemented at least partially in parallel with one or more other program modules. Generally, program modules include routines, programs, components and/or data structures that perform particular tasks and/or implement particular abstract data types. Moreover, the aforedescribed computer-implemented methods can be practiced with other computer system configurations, including single-processor and/or multiprocessor computer systems, mini-computing devices, mainframe computers, as well as computers, hand-held computing devices (e.g., PDA, phone), and/or microprocessor-based or programmable consumer and/or industrial electronics. The illustrated aspects can also be practiced in distributed computing environments in which tasks are performed by remote processing devices that are linked through a communications network. However, one or more, if not all aspects of the one or more example embodiments described herein can be practiced on stand-alone computers. In a distributed computing environment, program modules can be located in both local and remote memory storage devices.

As used in this application, the terms "component," "system," "platform" and/or "interface" can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form. As used herein, the terms "example" and/or "exemplary" are utilized to mean serving as an example, instance, or illustration. For the avoidance of doubt, the subject matter described herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

As it is employed in the subject specification, the term "processor" can refer to substantially any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular and quantum-dot based transistors, switches and/or gates, in order to optimize space usage and/or to enhance performance of related equipment. A processor can be implemented as a combination of computing processing units.

Herein, terms such as "store," "storage," "data store," data storage," "database," and substantially any other information storage component relevant to operation and functionality of a component are utilized to refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory and/or nonvolatile random-access memory (RAM) (e.g., ferroelectric RAM (FeRAM). Volatile memory can include RAM, which can act as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein are intended to include, without being limited to including, these and/or any other suitable types of memory.

What has been described above includes mere examples of systems and computer-implemented methods. It is, of course, not possible to describe every conceivable combination of components and/or computer-implemented methods for purposes of describing the one or more example embodiments, but one of ordinary skill in the art can recognize that many further combinations and/or permutations of the one or more example embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the various embodiments can use the phrases "an embodiment," "various embodiments," "one or more example embodiments" and/or "some embodiments," each of which can refer to one or more of the same or different embodiments.

The descriptions of the various embodiments have been presented for purposes of illustration but are not intended to be exhaustive or limited to the embodiments described herein. Many modifications and variations will be apparent to those of ordinary skill in the art without departing from the scope and spirit of the described embodiments. The terminology used herein was chosen to best explain the principles of the embodiments, the practical application and/or technical improvement over technologies found in the marketplace, and/or to enable others of ordinary skill in the art to understand the embodiments described herein.

## Claims

1. A computer-implemented method, comprising:

   executing, by a system operatively coupled to a processor, a comparison of a first molecular fingerprint, comprising first molecular structure data of a first molecular structure, to a second molecular fingerprint, comprising second molecular structure data of a second molecular structure; and
   generating, by the system, display data for visualizing a similarity visual illustrating representations of the first molecular structure, the second molecular structure, and a structural similarity score resulting from the comparison.

2. The computer-implemented method of claim 1, wherein the representations comprise an edge, corresponding to the structural similarity score, extending between a pair of nodes, corresponding to the first molecular structure and the second molecular structure.

3. The computer-implemented method of claim 1 or claim 2, further comprising:
   applying, by the system, a first property of the structural similarity score as a first visual modification of the edge and that applies a second property of the first molecular structure as a second visual modification of the respective node of the first molecular structure.

4. The computer-implemented method of claim 1, claim 2 or claim 3, further comprising:

> evaluating, by the system, identification metadata associated with the first molecular fingerprint or the second molecular fingerprint; and generating, by the system, based on the evaluating, the similarity visual comprising a visualization aspect that visually differentiates nodes, corresponding to the first molecular fingerprint or the second molecular fingerprint, from one another.

5. The computer-implemented method of any preceding claim, further comprising:
generating, by the system, the similarity visual being a cloud-type visual based on the display data, comprising a first generation of edges, representing structural similarity scores, including the structural similarity score, extending between a set of nodes representing a set of molecular structures of a library datastore, including a primary node representing the first molecular structure and a secondary node representing the second molecular structure.

6. The computer-implemented method of claim 5, further comprising:

> redistributing, by the system, a portion of the set of nodes, including the primary node, based on selection, at a graphical user interface displaying the similarity visual, of a classification filtering option corresponding to the first molecular structure; and
> generating, by the system, a set of similarity scores between nodes of the portion of the set of nodes resulting from the filtering.

7. A computer program for facilitating a process for visualizing and comparing molecular structures, the computer program having program instructions executable by a processor to cause the processor to carry out the method steps of any of the preceding claims.

8. A computer program product comprising a computer readable storage medium within which is embodied the computer program of claim 7.

9. A system comprising a memory within which is stored the computer program of claim 7, and a processor configured to execute the program instructions of the computer program stored in the memory.

SCIENTIFIC INSTRUMENT MODULE 100

FIRST (MOLECULAR
STRUCTURE DATA
IDENTIFICATION)
LOGIC 102

SECOND
(COMPARING)
LOGIC
104

THIRD
(DISPLAYING)
LOGIC
106

FOURTH
(FILTERING)
LOGIC
108

**FIG. 1**

200

PERFORM FIRST OPERATIONS:
IDENTIFYING MOLECULAR
STRUCTURE DATA
202

PERFORM SECOND OPERATIONS:
COMPARING MOLECULAR
FINGERPRINTS TO ONE ANOTHER
204

PERFORM THIRD OPERATIONS:
DISPLAYING THE COMPARISON
INCLUDING A STRUCTURAL
SIMILARITY SCORE
206

PERFORM FOURTH OPERATIONS:
FILTERING VARIOUS PARAMETERS
TO CUSTOMIZE A DISPLAYED MN
CLOUD VISUAL
208

# FIG. 2

GUI
300

DISPLAY REGION
302

DATA ANALYSIS REGION
304

SCIENTIFIC
INSTRUMENT
CONTROL REGION
306

SETTING REGION
308

**FIG. 3**

EP 4 712 085 A1

COMPUTING DEVICE 400

| PROCESSOR 402 | BATTERY/POWER CIRCUITRY 408 |
|---|---|
| STORAGE DEVICE 404 | DISPLAY DEVICE 410 |
| INTERFACE DEVICE 406 | OTHER I/O DEVICES 412 |

**FIG. 4**

FIG. 5

FIG. 6

FIG. 7

LIBRARY 635 OF MOLECULAR STRUCTURAL CONTENT 634

800

FP: 513

FP: 1057

FP: 1380

FP: 294

FP: 1095

FP: 682

FP: 378

FP: 1967

FP: 80

FP: 1745

FP: 1876

FP: 789

FP: 278

FP: 1205

FP: 725

FP: 1114

FP: 1694

FP: 1855

802

FIG. 8

FIG. 9

EP 4 712 085 A1

FIG. 10

FIG. 11

FIG. 12

## INTERACTIVE PROPERTY CUSTOMIZATION GUI 1300

<u>1302</u>

GENERAL PARAMETERS

- Number of Connections

-Number of Nodes

-Structural Similarity Score Range

<u>1304</u>

SIMILARITY SCORE BASIS

-Cosine

-HighChem

-NIST

-Tanimoto

<u>1306</u>

MULTI-CLASS CLASSIFICATIONS OR HEIRARCHICAL CLASSIFICATIONS

-First Classification

-Second Classification

-Third Classification

-Fourth Classification

<u>1308</u>

VISUALIZATION

-Edge Thickness

-Edge Color

-Edge Length

-Node Color

-Node Patterning

-Node Border Thickness

-Node Border Color

-Node Size

<u>1310</u>

NODE ION VISUALIZATION

-Ions M

-Ions N

-Ions O

EP 4 712 085 A1

# FIG. 13

1400

EXECUTING, BY A SYSTEM OPERATIVELY COUPLED TO A PROCESSOR, A COMPARISON OF A FIRST MOLECULAR FINGERPRINT, COMPRISING FIRST MOLECULAR STRUCTURE DATA OF A FIRST MOLECULAR STRUCTURE, TO A SECOND MOLECULAR FINGERPRINT, COMPRISING SECOND MOLECULAR STRUCTURE DATA OF A SECOND MOLECULAR STRUCTURE.

1402

IS THERE ADDITIONAL MOLECULAR STRUCTURE DATA TO COMPARE AGAINST?

YES

1404

NO

GENERATING, BY THE SYSTEM, DISPLAY DATA FOR VISUALIZING A SIMILARITY VISUAL ILLUSTRATING REPRESENTATIONS OF THE FIRST MOLECULAR STRUCTURE, THE SECOND MOLECULAR STRUCTURE, AND A STRUCTURAL SIMILARITY SCORE RESULTING FROM THE COMPARISON.

1406

**FIG. 14**

1500

| 1502 | OBTAINING, BY A SYSTEM OPERATIVELY COUPLED TO A PROCESSOR, MOLECULAR STRUCTURE DATA FOR PROCESSING. |

1504 — GENERATING, BY THE SYSTEM, A FIRST MOLECULAR FINGERPRINT BASED ON A FIRST MOLECULAR STRUCTURE, WHEREIN THE FIRST MOLECULAR FINGERPRINT IS UNIQUE TO THE FIRST MOLECULAR STRUCTURE AND HAS IDENTIFICATION METADATA, UNIQUE TO THE FIRST MOLECULAR FINGERPRINT, ASSOCIATED THEREWITH.

1506 — GENERATING, BY THE SYSTEM, A STRUCTURAL SIMILARITY SCORE BASED ON SUB-COMPARISONS OF FIRST FINGERPRINT BITS THAT ARE COMMON TO EACH OF THE FIRST MOLECULAR FINGERPRINT AND THE SECOND MOLECULAR FINGERPRINT, AND OF SECOND FINGERPRINT BITS THAT ARE UNCOMMON, TO EACH OF THE FIRST MOLECULAR FINGERPRINT AND THE SECOND MOLECULAR FINGERPRINT.

1508 — EXECUTING, BY A SYSTEM OPERATIVELY COUPLED TO A PROCESSOR, A COMPARISON OF THE FIRST MOLECULAR FINGERPRINT, COMPRISING FIRST MOLECULAR STRUCTURE DATA OF THE FIRST MOLECULAR STRUCTURE, TO A SECOND MOLECULAR FINGERPRINT, COMPRISING SECOND MOLECULAR STRUCTURE DATA OF A SECOND MOLECULAR STRUCTURE.

YES

1510 — IS THERE ADDITIONAL MOLECULAR STRUCTURE DATA TO COMPARE AGAINST?

NO

1512 — GENERATING, BY THE SYSTEM, DISPLAY DATA FOR VISUALIZING A SIMILARITY VISUAL ILLUSTRATING REPRESENTATIONS OF THE FIRST MOLECULAR STRUCTURE, THE SECOND MOLECULAR STRUCTURE, AND THE STRUCTURAL SIMILARITY SCORE RESULTING FROM THE COMPARISON.

1514 — GENERATING, BY THE SYSTEM, THE SIMILARITY VISUAL WHEREIN THE REPRESENTATIONS COMPRISE AN EDGE, CORRESPONDING TO THE STRUCTURAL SIMILARITY SCORE, EXTENDING BETWEEN A PAIR OF NODES, CORRESPONDING TO THE FIRST MOLECULAR STRUCTURE AND THE SECOND MOLECULAR STRUCTURE.

A

**FIG. 15**

1500

A

1515 — GENERATING, BY THE SYSTEM, THE SIMILARITY VISUAL COMPRISING A TWO-DIMENSIONAL REPRESENTATION OF THE FIRST MOLECULAR STRUCTURE AND THE SECOND MOLECULAR STRUCTURE THAT ARE MOVEABLE RELATIVE TO ONE ANOTHER AND SIZE ADJUSTABLE RELATIVE TO ONE ANOTHER.

1516 — GENERATING, BY THE SYSTEM, GENERATING, BY THE SYSTEM, THE SIMILARITY VISUAL BEING A CLOUD-TYPE VISUAL BASED ON THE DISPLAY DATA, COMPRISING A FIRST GENERATION OF EDGES, REPRESENTING STRUCTURAL SIMILARITY SCORES, INCLUDING THE STRUCTURAL SIMILARITY SCORE, EXTENDING BETWEEN A SET OF NODES REPRESENTING A SET OF MOLECULAR STRUCTURES OF A LIBRARY DATASTORE, INCLUDING A PRIMARY NODE REPRESENTING THE FIRST MOLECULAR STRUCTURE AND A SECONDARY NODE REPRESENTING THE SECOND MOLECULAR STRUCTURE.

1518 — EVALUATING, BY THE SYSTEM, IDENTIFICATION METADATA ASSOCIATED WITH THE FIRST MOLECULAR FINGERPRINT OR THE SECOND MOLECULAR FINGERPRINT.

1520 — GENERATING, BY THE SYSTEM, BASED ON THE EVALUATING, THE SIMILARITY VISUAL COMPRISING A VISUALIZATION ASPECT THAT VISUALLY DIFFERENTIATES NODES, CORRESPONDING TO THE FIRST MOLECULAR FINGERPRINT OR THE SECOND MOLECULAR FINGERPRINT, FROM ONE ANOTHER.

1522 — APPLYING, BY THE SYSTEM, A FIRST PROPERTY OF THE STRUCTURAL SIMILARITY SCORE AS A FIRST VISUAL MODIFICATION OF THE EDGE AND THAT APPLIES A SECOND PROPERTY OF THE FIRST MOLECULAR STRUCTURE AS A SECOND VISUAL MODIFICATION OF THE RESPECTIVE NODE OF THE FIRST MOLECULAR STRUCTURE.

1524 — ADJUSTING, BY THE SYSTEM, AT LEAST ONE OF THE FIRST VISUAL MODIFICATION OR THE SECOND VISUAL MODIFICATION BASED ON A SELECTION, AT A GRAPHICAL USER INTERFACE DISPLAYING THE SIMILARITY VISUAL, FROM A CLASS OF PROPERTIES COMPRISING PROPERTIES OTHER THAN THE RESPECTIVE ONE OF THE FIRST PROPERTY OR THE SECOND PROPERTY CORRESPONDING TO THE AT LEAST ONE OF THE FIRST VISUAL MODIFICATION OR THE SECOND VISUAL MODIFICATION.

1526 — REDISTRIBUTING, BY THE SYSTEM, A PORTION OF THE SET OF NODES, INCLUDING THE PRIMARY NODE, BASED ON SELECTION, AT A GRAPHICAL USER INTERFACE DISPLAYING THE SIMILARITY VISUAL, OF A CLASSIFICATION FILTERING OPTION CORRESPONDING TO THE FIRST MOLECULAR STRUCTURE.

1528 — GENERATING, BY THE SYSTEM, A SET OF SIMILARITY SCORES BETWEEN NODES OF THE PORTION OF THE SET OF NODES RESULTING FROM THE FILTERING.

**FIG. 16**

1700

REMOTE
COMPUTING
DEVICE
1740

PROCESSING
DEVICE
1702

STORAGE
DEVICE
1704

INTERFACE
DEVICE
1706

1708

SERVICE LOCAL
COMPUTING
DEVICE
1730

PROCESSING
DEVICE
1702

STORAGE
DEVICE
1704

INTERFACE
DEVICE
1706

SCIENTIFIC
INSTRUMENT
1710

PROCESSING
DEVICE
1702

STORAGE
DEVICE
1704

INTERFACE
DEVICE
1706

1708

1708

1708

USER LOCAL
COMPUTING
DEVICE
1720

PROCESSING
DEVICE
1702

STORAGE
DEVICE
1704

INTERFACE
DEVICE
1706

1708

1708

1712

**FIG. 17**

**FIG. 18**

FIG. 19

| | Europäisches Patentamt |
|---|---|
| | European Patent Office |
| | Office européen des brevets |

# EUROPEAN SEARCH REPORT

**Application Number**

EP 25 19 9592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCALFANI VINCENT F. ET AL: "Visualizing chemical space networks with RDKit and NetworkX", JOURNAL OF CHEMINFORMATICS, vol. 14, no. 1, 28 December 2022 (2022-12-28), XP093355830, London, UK ISSN: 1758-2946, DOI: 10.1186/s13321-022-00664-x Retrieved from the Internet: URL:https://link.springer.com/article/10.1186/s13321-022-00664-x/fulltext.html> * p. 1 p. 6 p. 11; figures 1, 6 * | 1-9 | INV. G16B40/10 G16B45/00 G16C20/40 G16C20/80 |
| X | ZHANG BIJUN ET AL: "Comparison of bioactive chemical space networks generated using substructure- and fingerprint-based measures of molecular similarity", JOURNAL OF COMPUTER-AIDED MOLECULAR DESIGN, SPRINGER NETHERLANDS, NL, vol. 29, no. 7, 7 June 2015 (2015-06-07), pages 595-608, XP035507406, ISSN: 0920-654X, DOI: 10.1007/S10822-015-9852-5 [retrieved on 2015-06-07] * p. 595 p. 601; figure 1 * | 1-9 | |

**TECHNICAL FIELDS SEARCHED (IPC)**

G16B
G16C

-/--

The present search report has been drawn up for all claims

| Place of search | Date of completion of the search | Examiner |
|---|---|---|
| Munich | 29 January 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

**Application Number**

EP 25 19 9592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VOGT MARTIN ET AL: "Lessons learned from the design of chemical space networks and opportunities for new applications", JOURNAL OF COMPUTER-AIDED MOLECULAR DESIGN, SPRINGER NETHERLANDS, NL, vol. 30, no. 3, 5 March 2016 (2016-03-05), pages 191-208, XP035650306, ISSN: 0920-654X, DOI: 10.1007/S10822-016-9906-3 [retrieved on 2016-03-05] * p. 192 p. 195 p. 201 p. 204 p. 207; figures 1, 11, 15 * | 1-9 | |
| X | WU MENGJUN ET AL: "Design of chemical space networks on the basis of Tversky similarity", JOURNAL OF COMPUTER-AIDED MOLECULAR DESIGN, SPRINGER NETHERLANDS, NL, vol. 30, no. 1, 22 December 2015 (2015-12-22), pages 1-12, XP035834623, ISSN: 0920-654X, DOI: 10.1007/S10822-015-9891-Y [retrieved on 2015-12-22] * p. 1 p. 3 p. 7; figure 3 * | 1-9 | TECHNICAL FIELDS SEARCHED (IPC) |

-/--

The present search report has been drawn up for all claims

| Place of search | Date of completion of the search | Examiner |
|---|---|---|
| Munich | 29 January 2026 | Mühlbauer, Max |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

**page 2 of 3**

Europäisches
Patentamt

European
Patent Office

Office européen
des brevets

## EUROPEAN SEARCH REPORT

**Application Number**

EP 25 19 9592

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARIKO I ITO ET AL: "Weighted Network Analysis of Biologically Relevant Chemical Spaces", ARXIV.ORG, CORNELL UNIVERSITY LIBRARY, 201 OLIN LIBRARY CORNELL UNIVERSITY ITHACA, NY 14853, 13 November 2019 (2019-11-13), XP081531476, * p. 1 * ----- | 1-9 | |
| | | | TECHNICAL FIELDS SEARCHED (IPC) |

The present search report has been drawn up for all claims

| Place of search | Date of completion of the search | Examiner |
|---|---|---|
| Munich | 29 January 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................
& : member of the same patent family, corresponding document

EPO FORM 1503 03.82 (P04C01)